(19) Europäisches Patentamt
European Patent Office
Office européen des brevets

(11) EP 1 625 129 B1

(12) EUROPÄISCHE PATENTSCHRIFT

(45) Veröffentlichungstag und Bekanntmachung des Hinweises auf die Patenterteilung:
**05.03.2008 Patentblatt 2008/10**

(21) Anmeldenummer: **04729875.7**

(22) Anmeldetag: **28.04.2004**

(51) Int Cl.:
***C07D 487/04*** *(2006.01)* ***A61K 31/517*** *(2006.01)*
***A61P 31/12*** *(2006.01)*

(86) Internationale Anmeldenummer:
**PCT/EP2004/004456**

(87) Internationale Veröffentlichungsnummer:
**WO 2004/099212 (18.11.2004 Gazette 2004/47)**

(54) **HETEROCYCLYL-SUBSTITUIERTE DIHYDROCHINAZOLINE UND IHRE VERWENDUNG ALS ANTIVIRALE MITTEL**

HETEROCYCLYL-SUBSTITUTED DIHYDROQUINAZOLINES AND USE THEREOF AS AN ANTIVIRAL AGENT

DIHYDROQUINAZOLINES SUBSTITUEES PAR HETEROCYCLYLE ET LEUR UTILISATION COMME ANTIVIRAUX

(84) Benannte Vertragsstaaten:
**DE ES FR GB IT**

(30) Priorität: **09.05.2003 DE 10320780**

(43) Veröffentlichungstag der Anmeldung:
**15.02.2006 Patentblatt 2006/07**

(73) Patentinhaber: **Bayer HealthCare AG**
**51368 Leverkusen (DE)**

(72) Erfinder:
- **WUNBERG, Tobias**
  **42699 Solingen (DE)**
- **BAUMEISTER, Judith**
  **42277 Wuppertal (DE)**
- **JESKE, Mario**
  **42699 Solingen (DE)**
- **NELL, Peter**
  **42115 Wuppertal (DE)**
- **NIKOLIC, Susanne**
  **40789 Monheim (DE)**
- **SÜSSMEIER, Frank**
  **42277 Wuppertal (DE)**
- **ZIMMERMANN, Holger**
  **42113 Wuppertal (DE)**
- **GROSSER, Rolf**
  **51375 Leverkusen (DE)**
- **HENNINGER, Kerstin**
  **42115 Wuppertal (DE)**
- **HEWLETT, Guy**
  **42327 Wuppertal (DE)**
- **KELDENICH, Jörg**
  **42113 Wuppertal (DE)**
- **LANG, Dieter**
  **42553 Velbert (DE)**

(56) Entgegenhaltungen:
**EP-A- 1 201 765**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 625 129 B1

## Beschreibung

Die Erfindung betrifft Heterocyclyl-substituierte Dihydrochinazoline und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

Die Synthese von Dihydrochinazolinen ist beschrieben in Saito T., et al. Tetrahedron Lett., 1996, 37, 209-212 und in Wang F., et al. Tetrahedron Lett., 1997, 38, 8651-8654.

4-Anilinochinazoline mit antiviraler Wirkung gegen Cytomegaloviren werden in EP 1 201 765 beschrieben.

Auf dem Markt sind zwar strukturell andersartige antiviral wirkende Mittel vorhanden, es kann aber regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen Heterocyclyl-substituierte Dihydrochinazoline antiviral wirksam sind.

Gegenstand der Erfindung sind Verbindungen der Formel

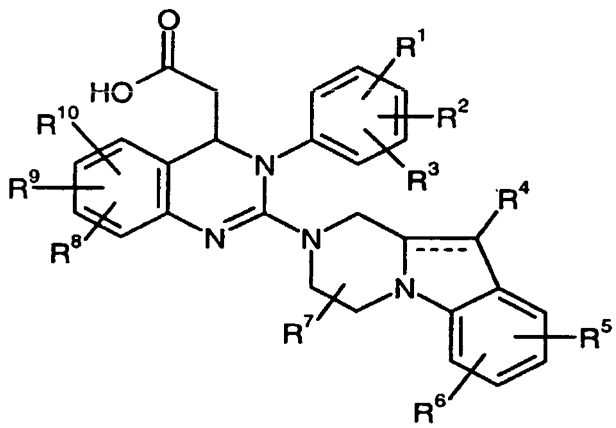

(I),

in welcher

........ für eine Einfach- oder Doppelbindung steht,

$R^1$ für Wasserstoff, Amino, Alkyl, Alkoxy, Alkylamino, Alkylthio, Cyano, Halogen, Nitro oder Trifluormethyl steht,

$R^2$ für Wasserstoff, Alkyl, Alkoxy, Alkylthio, Cyano, Halogen, Nitro oder Trifluormethyl steht,

$R^3$ für Amino, Alkyl, Alkoxy, Alkylamino, Alkylthio, Cyano, Halogen, Nitro, Trifluormethyl, Alkylsulfonyl oder Alkylaminosulfonyl steht

oder

einer der Reste $R^1$, $R^2$ und $R^3$ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,

$R^4$ für Wasserstoff oder Alkyl steht,

$R^5$ für Wasserstoff, Alkyl, Alkoxy, Formyl, Carboxy, Alkylcarbonyl, Alkoxycarbonyl, Trifluormethyl, Halogen, Cyano, Hydroxy, Amino, Alkylamino, Aminocarbonyl oder Nitro steht,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,

$R^6$ für Wasserstoff, Alkyl, Alkoxy, Formyl, Carboxy, Alkylcarbonyl, Alkoxycarbonyl, Trifluormethyl, Halogen, Cyano, Hydroxy, Amino, Alkylamino, Aminocarbonyl oder Nitro steht,

worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl

oder

$R^5$ und $R^6$ zusammen mit den Kohlenstoffatomen, an die sie gebunden sind, ein 1,3-Dioxolan, einen Cyclopentan-Ring oder einen Cyclohexan-Ring bilden,

$R^7$ für Wasserstoff oder Alkyl steht,

$R^8$ für Wasserstoff, Alkyl, Alkoxy, Alkylamino, Alkylthio, Formyl, Carboxy, Aminocarbonyl, Alkylaminocarbonyl, Alkylcarbonyl, Alkoxycarbonyl, Trifluormethyl, Halogen, Cyano, Hydroxy, Nitro oder einen über Stickstoff gebundenen 5- bis 7-gliedrigen Heterocyclus steht,

$R^9$ für Wasserstoff, Alkyl, Alkoxy, Alkylthio, Formyl, Carboxy, Alkylcarbonyl, Alkoxycarbonyl, Trifluormethyl, Halogen, Cyano, Hydroxy oder Nitro steht
und

$R^{10}$ für Wasserstoff, Alkyl, Alkoxy, Alkylthio, Formyl, Carboxy, Alkylcarbonyl, Alkoxycarbonyl, Trifluormethyl, Halogen, Cyano, Hydroxy oder Nitro steht,

und ihre Salze, ihre Solvate und die Solvate ihrer Salze.

Erfindungsgemäße Verbindungen sind die Verbindungen der Formel (I) und deren Salze, Solvate und Solvate der Salze, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylthio, Alkylamino, Alkylaminocarbonyl, Alkylcarbonyl, Alkylsulfonyl, Alkylaminosulfonyl und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert.*-Butyl, n-Pentyl und n-Hexyl.

Alkoxy steht beispielhaft und vorzugsweise für Methoxy, Ethoxy, n-Propoxy, Isopropoxy, *tert*.-Butoxy, n-Pentoxy und n-Hexoxy.

Alkylthio steht beispielhaft und vorzugsweise für Methylthio, Ethylthio, n-Propylthio, Isopropylthio, tert.-Butylthio, n-Pentylthio und n-Hexylthio.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*.-Butylamino, n-Pentylamino, n-Hexylamino, *N,N*-Dimethylamino, *N,N* Diethylamino, *N*-Ethyl-*N*-methylamino, *N*-Methyl-*N*-n-propylamino, *N*-Isopropyl-*N*-n-propylamino, *N-tert.*-Butyl-*N*-methylamino, *N*-Ethyl-*N*-n-pentylamino und *N*-n-Hexyl-*N*-methylamino. $C_1$-$C_3$-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*.-Butylaminocarbonyl, n-Pentylaminocarbonyl, n-Hexylaminocarbonyl, *N, N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N-tert.*-Butyl-*N*-methylaminocarbonyl, *N*-Ethyl-*N*-n-pentylaminocarbonyl und *N*-n-Hexyl-*N*-methylaminocarbonyl. $C_1$-$C_3$-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Alkylsulfonyl steht beispielhaft und vorzugsweise für Methylsulfonyl, Ethylsulfonyl, n-Propylsulfonyl, Isopropylsulfonyl, *tert*.-Butylsulfonyl, n-Pentylsulfonyl und n-Hexylsulfonyl.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, Isopropylaminosulfonyl, *tert*.-Butylaminosulfonyl, n-Pentylaminosulfonyl, n-Hexylaminosulfonyl, *N,N* Dimethylaminosulfonyl, *N,N*-Diethylaminosulfonyl, *N*-Ethyl-*N*-methylanünosulfonyl, *N*-Methyl-*N*-n-propylaminosulfonyl, *N*-Isopropyl-*N*-n-propylaminosulfonyl, *N-tert.-Butyl-N*-methylaminosulfonyl, *N*-Ethyl-*N*-n-pentylaminosulfonyl und N-n-Hexyl-*N*-methylaminosulfonyl. $C_1$-$C_3$-Alkylaminosulfonyl steht beispielsweise für einen Monoalkylaminosulfonylrest mit 1 bis 3 Kohlenstoffatomen oder für einen Dialkylaminosulfonylrest mit jeweils 1 bis 3 Kohlenstoffatomen pro Alkylsubstituent.

Alkylcarbonyl steht beispielhaft und vorzugsweise für Acetyl und Propanoyl.

Alkoxycarbonyl steht beispielhaft und vorzugsweise für Methoxycarbonyl, Ethoxycarbonyl, n-Propoxycarbonyl, Isopropoxycarbonyl, *tert.*-Butoxycarbonyl, n-Pentoxycarbonyl und n-Hexoxycarbonyl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Über Stickstoff gebundener 5- bis 7-gliedriger Heterocyclus steht für einen über Stickstoff gebundenen monocyclischen, nicht-aromatischen heterocyclischen Rest mit in der Regel 5 bis 7, vorzugsweise 5 oder 6 Ringatomen und bis zu 2, vorzugsweise bis zu einem zusätzlichen Heteroatom und/oder Heterogruppe aus der Reihe N, O, S, SO, $SO_2$. Der Heterocyclus kann gesättigt oder teilweise ungesättigt sein. Bevorzugt sind 5- bis 6-gliedrige, monocyclische gesättigte Heterocyclen mit bis zu einem zusätzlichen Heteroatom aus der Reihe O, N und S, wie beispielhaft und vorzugsweise Pyrrolidinyl, Piperidinyl, Piperazinyl, Morpholinyl und Thiomorpholinyl.

Halogen steht für Fluor, Chlor, Brom und Jod, bevorzugt Fluor und Chlor.

Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90 % verstanden wird (> 90 % ee).

Bevorzugt sind solche Verbindungen der Formel (I), in welchen

........ für eine Einfach- oder Doppelbindung steht,

$R^1$ für Wasserstoff, $C_1$-$C_6$-Alkyl, $C_1$-$C_6$-Alkoxy, $C_1$-$C_6$-Alkylthio, Fluor oder Chlor steht,

$R^2$ für Wasserstoff, $C_1$-$C_6$-Alkyl, $C_1$-$C_6$-Alkoxy, $C_1$-$C_6$-Alkylthio, Fluor oder Chlor steht,

$R^3$ für $C_1$-$C_4$-Alkyl, Cyano, Fluor, Chlor, Nitro oder Trifluormethyl steht,

oder
einer der Reste $R^1$, $R^2$ und $R^3$ für Wasserstoff, $C_1$-$C_3$-Alkyl, $C_1$-$C_3$-Alkoxy, Cyano, Halogen, Nitro oder Trifluormethyl steht und die anderen beiden zusammen mit den Kohlenstoffatomen, an die sie gebunden sind, einen Cyclopentan-Ring oder einen Cyclohexan-Ring bilden,

$R^4$ für Wasserstoff steht,

$R^5$ für Wasserstoff, $C_1$-$C_6$-Alkyl, $C_1$-$C_6$-Alkoxy, Carboxy, $C_1$-$C_6$-Alkylcarbonyl, $C_1$-$C_6$-Alkoxycarbonyl, Trifluormethyl,

Fluor, Chlor, Brom, Cyano, Hydroxy, Amino, $C_1$-$C_6$-Alkylamino oder Nitro steht,

$R^6$ für Wasserstoff, $C_1$-$C_6$-Alkyl, $C_1$-$C_6$-Alkoxy, Carboxy, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino oder Nitro steht

oder

$R^5$ und $R^6$ zusammen mit den Kohlenstoffatomen, an die sie gebunden sind, ein 1,3-Dioxolan bilden,

$R^7$ für Wasserstoff oder Methyl steht,

$R^8$ für $C_1$-$C_3$-Alkyl, $C_1$-$C_3$-Alkoxy, Carboxy, Aminocarbonyl, $C_1$-$C_3$-Alkylaminocarbonyl, Trifluormethyl, Fluor, Chlor, Cyano, Hydroxy oder Nitro steht,

$R^9$ für Wasserstoff, $C_1$-$C_3$-Alkyl, $C_1$-$C_3$-Alkoxy, Fluor, Chlor, Cyano oder Hydroxy steht und

$R^{10}$ für Wasserstoff, $C_1$-$C_3$-Alkyl, $C_1$-$C_3$-Alkoxy, Fluor, Chlor, Cyano oder Hydroxy steht.

Bevorzugt sind darunter besonders solche Verbindungen der Formel (I), in welchen

........ für eine Einfach- oder Doppelbindung steht,

$R^1$ für Wasserstoff, Methyl, Methoxy, Methylthio, Fluor oder Chlor steht,

$R^2$ für Wasserstoff steht,

$R^3$ für Methyl, Cyano, Fluor, Chlor, Nitro oder Trifluormethyl steht,

$R^4$ für Wasserstoff steht,

$R^5$ für Wasserstoff, Methyl, Methoxy, Fluor oder Chlor steht,

$R^6$ für Wasserstoff, Methyl, Methoxy, Fluor oder Chlor steht,

$R^7$ für Wasserstoff steht,

$R^8$ für Aminocarbonyl, Fluor, Chlor, Cyano oder Hydroxy steht,

$R^9$ für Wasserstoff steht und

$R^{10}$ für Wasserstoff steht.

Bevorzugt sind auch solche Verbindungen der Formel (I), in welchen ........ für eine Einfachbindung steht.

Bevorzugt sind auch solche Verbindungen der Formel (I), in weichen $R^1$ für Wasserstoff, Methyl, Methoxy oder Fluor steht.

Bevorzugt sind darunter besonders solche Verbindungen der Formel (I), in welchen $R^1$ für Methoxy steht.

Bevorzugt sind auch solche Verbindungen der Formel (I), in welchen $R^1$ über die ortho-Position zur Verknüpfungsstelle des Phenylrings an den Phenylring gebunden ist. Unter der Verknüpfungsstelle des mit den Resten $R^1$, $R^2$ und $R^3$ substituierten Phenylrings wird im Rahmen der vorliegenden Erfindung das gemäß Formel (I) mit einem der beiden Dihydrochinazolin-Stickstoffatome verknüpfte Kohlenstoffatom des Phenylrings verstanden.

Besonders bevorzugt sind solche Verbindungen der Formel (I), in welchen $R^1$ für Methoxy steht und $R^1$ über die ortho-Position zur Verknüpfungsstelle des Phenylrings an den Phenylring gebunden ist.

Bevorzugt sind auch solche Verbindungen der Formel (I), in welchen $R^2$ für Wasserstoff steht.

Bevorzugt sind auch solche Verbindungen der Formel (I), in welchen $R^3$ für Trifluormethyl, Chlor, Methyl, iso-Propyl oder *tert.*-Butyl steht.

Bevorzugt sind darunter besonders solche Verbindungen der Formel (I), in welchen $R^3$ für Trifluormethyl, Chlor

oder Methyl steht.

Bevorzugt sind auch solche Verbindungen der Formel (I), in welchen $R^1$ über die ortho-Position zur Verknüpfungsstelle des Phenylrings an den Phenylring gebunden ist und $R^3$ über die $R^1$ gegenüberliegende meta-Position zur Verknüpfungsstelle des Phenylrings an den Phenylring gebunden ist.

Besonders bevorzugt sind solche Verbindungen der Formel (I), in welchen $R^1$ über die ortho-Position zur Verknüpfungsstelle des Phenylrings an den Phenylring gebunden ist, $R^3$ für Trifluormethyl, Chlor oder Methyl steht und $R^3$ über die $R^1$ gegenüberliegende meta-Position zur Verknüpfungsstelle des Phenylrings an den Phenylring gebunden ist.

Bevorzugt sind auch solche Verbindungen der Formel (I), in welchen $R^4$ für Wasserstoff steht.

Bevorzugt sind auch solche Verbindungen der Formel (I), in welchen $R^5$ für Wasserstoff, Methyl, Methoxy, Fluor oder Chlor steht.

Bevorzugt sind auch solche Verbindungen der Formel (I), in welchen $R^6$ für Wasserstoff, Methyl, Methoxy oder Fluor steht.

Bevorzugt sind auch solche Verbindungen der Formel (I), in welchen $R^7$ für Wasserstoff steht.

Bevorzugt sind auch solche Verbindungen der Formel (I), in welchen $R^8$ für Fluor steht.

Besonders bevorzugt sind solche Verbindungen der Formel (I), in welchen $R^8$ für Fluor steht und $R^8$, wie in Formel

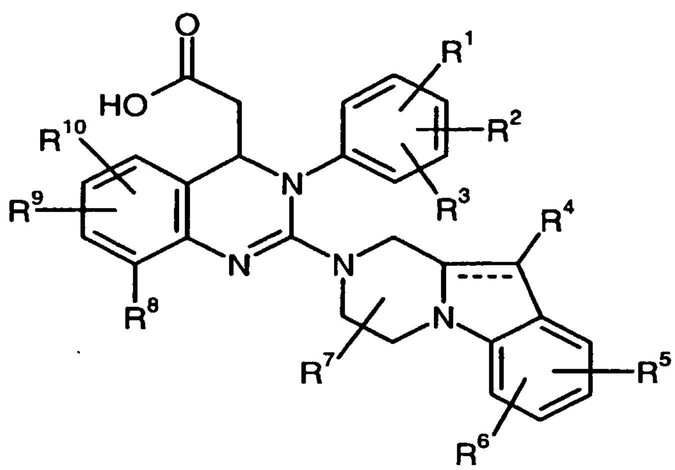

(Ia)

beschrieben, an den Aromaten des Dihydrochinazolines gebunden ist.

Bevorzugt sind auch solche Verbindungen der Formel (I), in welchen $R^9$ für Wasserstoff steht.

Bevorzugt sind darunter besonders solche Verbindungen der Formel (I), in welchen $R^{10}$ für Wasserstoff, Methyl oder Fluor steht.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei

nach Verfahren [A]
Verbindungen der Formel

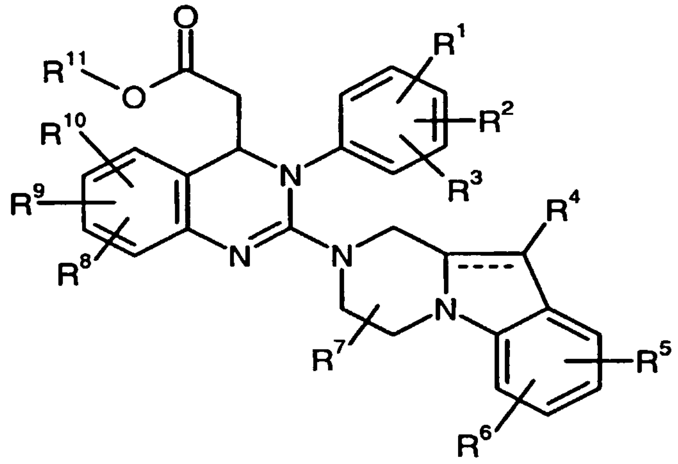

(II),

in welcher

| | |
|---|---|
| ......, $R^1$, $R^2$, $R^3$, $R^4$, $R^5$, $R^6$, $R^7$, $R^8$, $R^9$und $R^{10}$ | die oben angegebene Bedeutung haben und |
| $R^{11}$ | für Alkyl, bevorzugt Methyl oder Ethyl, steht, |

mit Basen umgesetzt werden,
oder
nach Verfahren [B]
Verbindungen der Formel

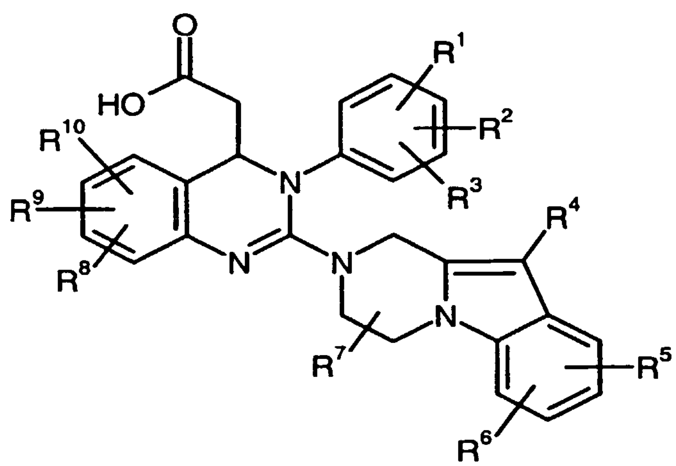

(Ib),

in welcher
$R^1$, $R^2$, $R^3$, $R^4$, $R^5$, $R^6$, $R^7$, $R^8$, $R^9$ und $R^{10}$ die oben angegebene Bedeutung haben,
durch Umsetzung mit Reduktionsmitteln zu Verbindungen der Formel

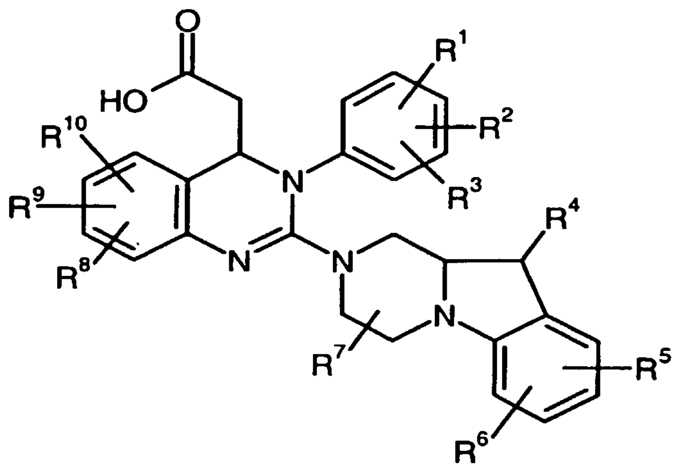

(Ic),

in welcher
$R^1$, $R^2$, $R^3$, $R^4$, $R^5$, $R^6$, $R^7$, $R^8$, $R^9$ und $R^{10}$ die oben angegebene Bedeutung haben,
umgesetzt werden.

Die Umsetzung nach Verfahren [A] erfolgt im Allgemeinen in inerten Lösungsmitteln, bevorzugt in einem Temperaturbereich von Raumtemperatur bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, gegebenenfalls in wässriger Lösung, bevorzugt ist Natriumhydroxid in Wasser.

Inerte Lösungsmittel sind beispielsweise Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert*.-Butanol, oder Gemischen von Lösungsmitteln, bevorzugt ist Dioxan oder Tetrahydrofuran.

Die Umsetzung nach Verfahren [B] erfolgt gegebenenfalls in inerten Lösungsmitteln, bevorzugt in einem Temperaturbereich von 0˚C bis Raumtemperatur bei Normaldruck.

Reduktionsmittel sind beispielsweise Zinn/Salzsäure, oder Hydrierungen mit Wasserstoff mit Katalysatoren wie Paladium-Kohle, Platin, Platinoxid, Raney-Nickel, Rh(acac)(cod)-2$PPh_3$, sowie Kombinationen von Hydriddonoren mit Säuren.

Hydriddonoren sind beispielsweise Natriumcyanoborhydrid, Kaliumborhydrid, $BH_3$·THF, $[F_3CC(O)O]_2BH$·THF oder Triethylsilan.

Säuren sind beispielsweise Carbonsäuren wie Essigsäure oder Trifluoressigsäure.

Bevorzugt ist Natriumcyanoborhydrid/Eisessig mit Eisessig als Lösungsmittel.

Inerte Lösungsmittel sind beispielsweise Alkohole wie Methanol, Ethanol oder iso-Propanol, Ether wie Diethylether, Tetrahydrofuran oder Dioxan, bevorzugt ist Ethanol.

Die Verbindungen der Formel (Ib) lassen sich nach Verfahren [A] herstellen.

Die Verbindungen der Formel (II) sind bekannt oder können hergestellt werden, indem Verbindungen der Formel

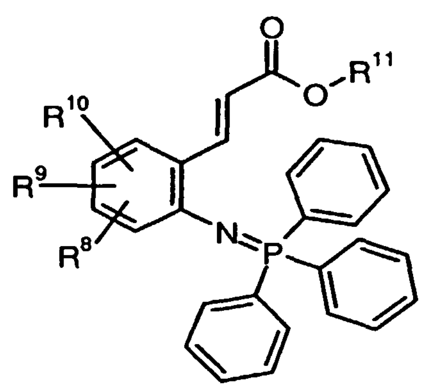

(III),

in welcher
$R^8$, $R^9$, $R^{10}$ und $R^{11}$ die oben angegebene Bedeutung haben,
in einer zweistufigen Reaktion zunächst mit Verbindungen der Formel

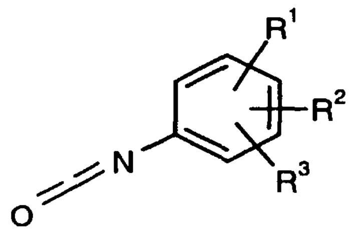
(IV),

in welcher
$R^1$, $R^2$ und $R^3$ die oben angegebene Bedeutung haben,
und anschließend mit Verbindungen der Formel

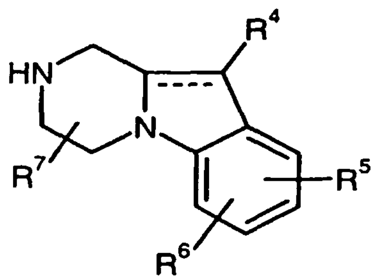
(V),

in welcher
....., $R^4$, $R^5$, $R^6$ und $R^7$ die oben angegebene Bedeutung haben,
umgesetzt werden.

Die Umsetzung erfolgt in beiden Stufen im allgemeinen in inerten Lösungsmitteln, bevorzugt in einem Temperaturbereich von Raumtemperatur bis 100°C bei Normaldruck. In der zweiten Stufe wird gegebenenfalls Kieselgel zu der Reaktionsmischung dazugegeben. Die Umsetzung erfolgt bevorzugt mit einer Aufarbeitung zwischen der ersten und der zweiten Stufe.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Essigsäureethylester, oder Gemischen von Lösungsmitteln, bevorzugt ist Methylenchlorid.

Die Verbindungen der Formel (IV) sind bekannt oder lassen sich nach bekannten Verfahren aus den entsprechenden Edukten synthetisieren.

Die Verbindungen der Formel (V) sind bekannt oder lassen sich nach bekannten Verfahren aus den entsprechenden Edukten synthetisieren (z.B. J. Chang-Fong, et al., Bioorg. Med. Chem. Lett. 2002, 12, 155-158; Y. Nakamura, et al., Org. Lett. 2002, 4, 2317-2320; L.D. Basanagoudar, et al., Ind. J. Chem. 1991, 30B, 1014-1017; S.B. Rajur, et al., Ind. J. Chem. 1989, 28B, 1065-1068; A.C. Cheng, et al., J. Org. Chem., 1982, 47, 5258-5262):

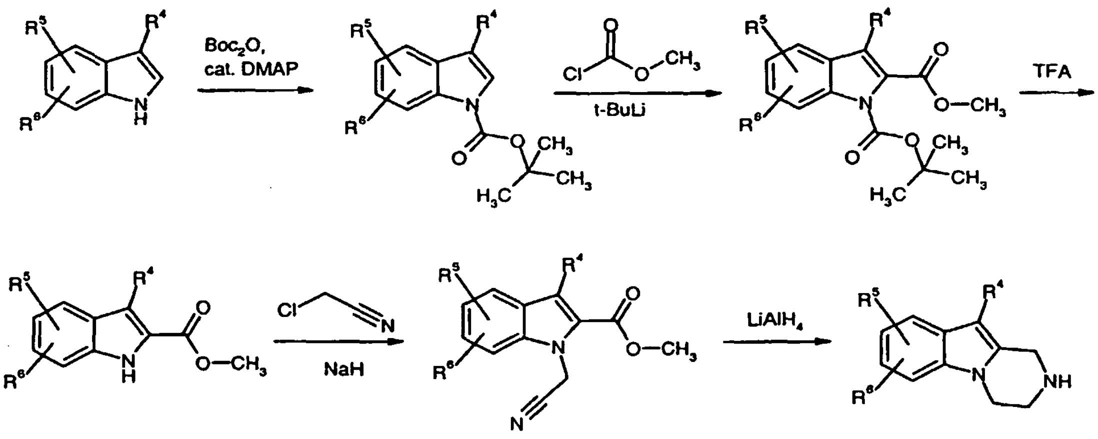


Die dafür benötigten Edukte sind bekannt oder lassen sich nach bekannten Verfahren aus den entsprechenden Edukten synthetisieren.

Die Verbindungen der Formel (III) sind bekannt oder können hergestellt werden, indem Verbindungen der Formel

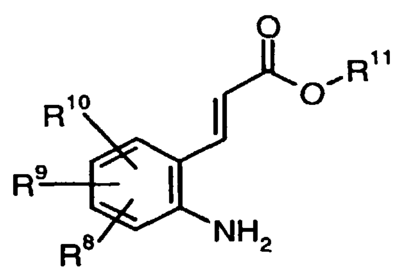

(VI),

in welcher

$R^8$, $R^9$, $R^{10}$ und $R^{11}$ die oben angegebene Bedeutung haben,

mit Triphenylphosphin und Tetrachlorkohlenstoff umgesetzt werden.

Die Umsetzung erfolgt im allgemeinen in inerten Lösungsmitteln, in Gegenwart einer Base, bevorzugt in einem Temperaturbereich von Raumtemperatur bis 50°C bei Normaldruck.

Inerte Lösungsmittel sind beispielsweise Ether wie Diethylether, Methyl-*tert.*-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, bevorzugt ist Acetonitril.

Basen sind beispielsweise Alkali- und Erdalkalicarbonate wie Cäsiumcarbonat, Natrium- oder Kaliumcarbonat oder Amine wie Triethylamin, Diisopropylethylamin, N-Methylmorpholin oder Pyridin, bevorzugt ist Triethylamin.

Die Verbindungen der Formel (VI) sind bekannt oder lassen sich nach bekannten Verfahren aus den entsprechenden Edukten synthetisieren, beispielsweise durch eine Heck-Reaktion oder eine Wittig-Horner-Reaktion nach folgenden Syntheseschemata:

Heck-Reaktion:

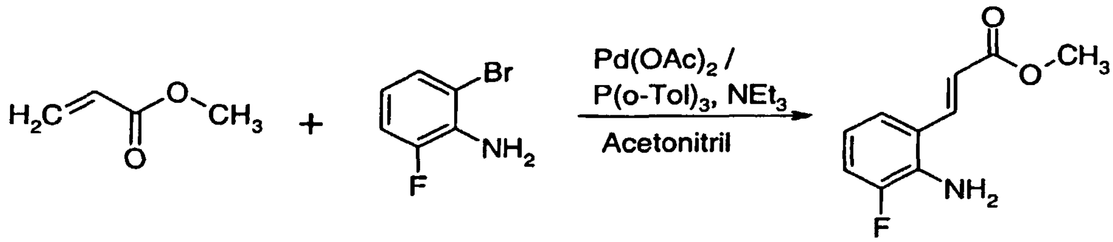


Wittig-Horner-Reaktion:

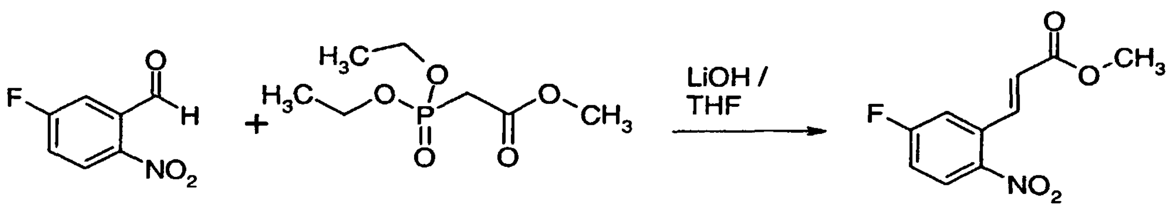


Die dafür benötigten Edukte sind bekannt oder lassen sich nach bekannten Verfahren aus den entsprechenden Edukten synthetisieren.

Die Herstellung der erfindungsgemäßen Verbindungen kann durch das folgende Syntheseschema verdeutlicht werden.

Syntheseschema:

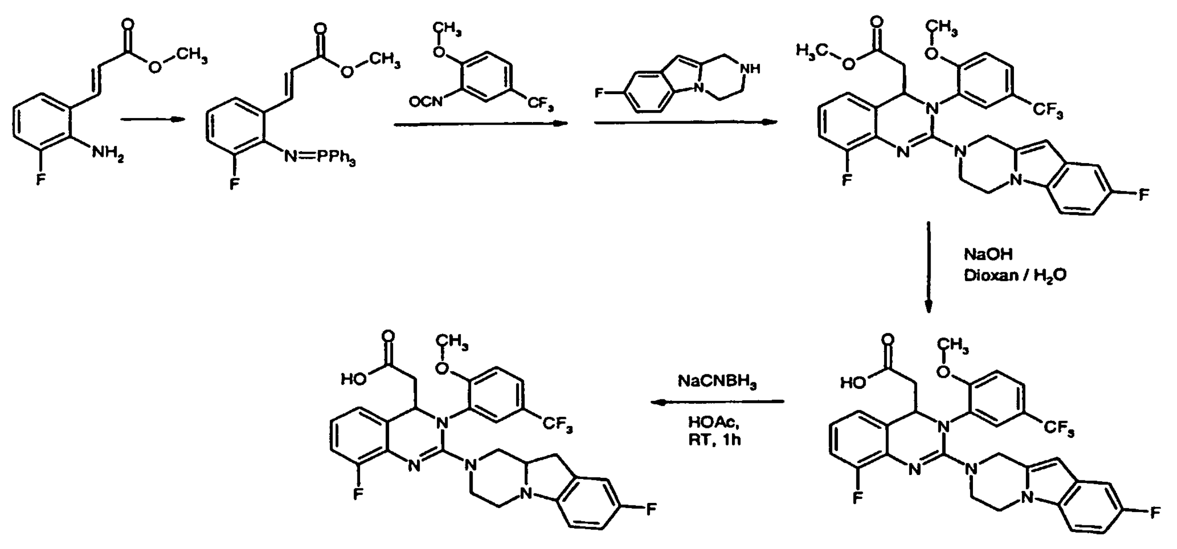


Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:

1) Behandlung und Prophylaxe von HCMV-Infektionen bei AmS-Patienten (Retinitis, Pneumonitis, gastrointestinale Infektionen).

2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

3) Behandlung und Prophylaxe von HCMV-Infektionen bei Neugeborenen und Kleinkindern.

4) Behandlung einer akuten HCMV-Infektion bei Schwangeren.

5) Behandlung der HCMV-Infektion bei immunsupprimierten Patienten bei Krebs und KrebsTherapie.

6) Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 50 mg/kg, vorzugsweise 0.1 bis 25 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

## A. Beispiele

### Abkürzungen:

| | |
|---|---|
| ca. | circa |
| BINAP | 2,2'-Bis(diphenylphosphino)-1,1'-binaphthyl |
| Boc | *tert*.-Butyloxycarbonyl |
| $CDCl_3$ | Deuterochloroform |
| DC | Dünnschichtchromatographie |
| DCI | direkte chemische Ionisation (bei MS) |
| DCM | Dichlormethan |
| DIEA | *N,N*-Diisopropylethylamin |
| DMSO | Dimethylsulfoxid |
| DMF | *N,N*-Dimethylformamid |
| d. Th. | der Theorie |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| H | Stunde |
| HPLC | Hochdruck-, Hochleistungsflüssigchromatographie |
| konz. | konzentriert |
| LC-MS | Flüssigchromatographie-gekoppelte Massenspektroskopie |
| LDA | Lithium-Diisopropylamid |
| MTBE | Methyl-*tert*.-butylether |
| min | Minuten |
| MS | Massenspektroskopie |
| NMR | Kernresonanzspektroskopie |
| Pd-C | Palladium auf Kohle |
| proz. | prozentig |
| RP-HPLC | Reverse Phase HPLC |
| RT | Raumtemperatur |
| $R_t$ | Retentionszeit (bei HPLC) |
| THF | Tetrahydrofuran |

**Allgemeine Methoden LC-MS und HPLC:**

**Methode 1 (analytische HPLC):** Säule: Kromasil C18 60 mm x 2 mm; Temperatur: 30°C; Fluss: 0.75 ml/min; Eluent A: 0.005 M $HClO_4$, Eluent B: Acetonitril; Gradient: → 0.5 min 98 %A, → 4.5 min 10 %A, → 6.5 min 10 %A.

**Methode 2 (präparative HPLC):** Säule: GromSil C18, 250 mm x 30 mm; Fluss: 50 ml/min; Laufzeit: 38 min; Eluent A: Wasser, Eluent B: Acetonitril, Gradient: 10 %B (3 min) → 90 %B (31 min) → 90 %B (34 min) → 10 %B (34.01 min); UV-Detektion: 210 nm.

**Methode 3 (LC-MS):** Säule: GromSil 120 ODS-4 HE, 50 mm x 2.0 mm, 3 µm; Eluent A: 11 Wasser + 1 ml 50%ige Ameisensäure, Eluent B: 11 Acetonitril + 1 ml 50 %ige Ameisensäure; Gradient: 0.0 min 100 %A → 0.2 min 100 %A → 2.9 min 30 %A → 3.1 min 10 %A → 4.5 min 10 %A; Ofen: 55°C; Fluss: 0.8 ml/min; UV-Detektion: 208-400 nm.

**Methode 4 (präparative HPLC, Enantiomerentrennung):** Säule: Chiraler Kieselgelselektor Packungsmaterial KBD 8361 (250 mm x 30 mm) basierend auf dem Selektor Poly(N-methacryloyl-L-leucin-1-menthylamid); Temperatur: 23°C; Eluent: Methyl-*tert*.-butylether; Fluss: 100 ml/min; Verbindung gelöst in MTBE/Essigsäureethylester (9: 1).

**Methode 5 (LC-MS):** Säule: GromSil 120 ODS-4 HE 50mm x 2 mm, 3.0 µm; Eluent A: Wasser + 500 µl 50 %ige Ameisensäure/l, Eluent B: Acetonitril + 500 µl 50 %ige Ameisensäure/l; Gradient: 0.0 min 0 %B → 2.9 min 70 %B → 3.1 min 90 %B → 4.5 min 90 %B; Ofen: 50°C; Fluss: 0.8 ml/min; UV-Detektion: 210 nm.

**Methode 6 (LC-MS):** Instrument: Micromass Quattro LCZ, HP1100; Säule: Symmetry C18, 50 mm x 2.1 mm, 3.5 µm; Eluent A: Acetonitril + 0.1% Ameisensäure, Eluent B: Wasser + 0.1 % Ameisensäure; Gradient: 0.0 min 10 %A → 4.0 min 90 %A → 6.0 min 90 %A; Ofen: 40°C; Fluss: 0.5ml/min; UV-Detektion: 208-400 nm.

**Methode 7 (LC-MS):** Säule: GromSil 120 ODS-4 HE, 50 mm x 2.0 mm, 3 µm; Eluent A: 11 Wasser + 1 ml 50 %ige Ameisensäure, Eluent B: 11 Acetonitril + 1 ml 50 %ige Ameisensäure; Gradient: 0.0 min 100 %A → 0.2 min 100 %A → 2.9 min 30 %A → 3.1 min 10 %A → 4.5 min 10 %A; Ofen: 55°C; Fluss: 0.8 ml/min; UV-Detektion: 208-400 nm.

**Methode 8 (LC-MS):** Gerätetyp MS: Micromass ZQ; Gerätetyp HPLC: Waters Alliance 2795; Säule: Merck Chromolith SpeedROD RP-18e 50 mm x 4.6mm; Eluent A: Wasser + 500 µl 50 %ige Ameisensäure/l; Eluent B: Acetonitril + 500 µl 50 %ige Ameisensäure/l; Gradient: 0.0 min 10 %B→ 3.0 min 95 %B→ 4.0 min 95 %B; Ofen: 35°C; Fluss: 0.0 min 1.0 ml/min→ 3.0 min 3.0 ml/min→ 4.0 min 3.0 ml/min; UV-Detektion: 210 nm.

**Methode 9 (präparative HPLC, Enantiomerentrennung):** Säule: Daicel-Kieselgelphase Chiralpak AD; Eluent: i-Hexan/Ethanol/Diethylamin (90/10/0.02; v/v/v).

**Ausgangsverbindungen**

**Arbeitsvorschrift [A]: Veresterung von 2-Nitrozimtsäuren mit Methanol**

517.7 mmol 2-Nitrozimtsäure werden in 600 ml Methanol vorgelegt und dann mit 20 Tropfen konzentrierter Schwefelsäure versetzt und für 72 Stunden unter Rückfluss erhitzt. Nach beendeter Reaktion (Reaktionskontrolle mittels DC) wird die Reaktionslösung mit einem Eisbad gekühlt. Die entstehenden Kristalle werden abgesaugt. Die Mutterlauge wird etwas eingeengt und die dabei entstehenden Kristalle werden abgesaugt. Beide Fraktionen werden vereinigt und aus Methanol bei RT umkristallisiert.

**Beispiel 1A**

(2E)-3-(2-Nitrophenyl)-propensäuremethylester

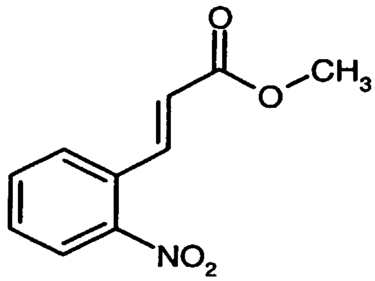

Ausgehend von 100.0 g (517.7 mmol) 2-Nitrozimtsäure werden nach der allgemeinen Arbeitsvorschrift [A] 72.6 g (68 % d. Th.) Produkt erhalten.
HPLC (Methode 1 ): $R_t$ = 4.21 min

**Allgemeine Arbeitsvorschrift [B]: Synthese von substituierten 2-Aminozimtsäurederivaten mittels Heck-Kupplung aus 2-halogensubstituierten Anilinen**

In einem Einhalskolben werden 1.0 Äquivalente eines Arylhalogenids mit 1.6 Äquivalenten Acrylsäuremethylester, 2.0 Äquivalenten Triethylamin, 0.03 Äquivalenten Palladium(II)acetat und 0.03 Äquivalenten Tri-o-tolylphosphin in Acetonitril vorgelegt (ca. IM-Lösung). Man lässt das Gemisch unter Rückfluss für 48 Stunden rühren. Nach beendeter Reaktion (Reaktionskontrolle mittels DC) wird das Lösemittel entfernt. Der Rückstand wird über Kieselgel mit Cyclohexan/Essigsäureethylester = 8:2 v/v chromatographisch gereinigt.

**Beispiel 2A**

(2E)-3-[2-Amino-3-fluorphenyl]-propensäuremethylester

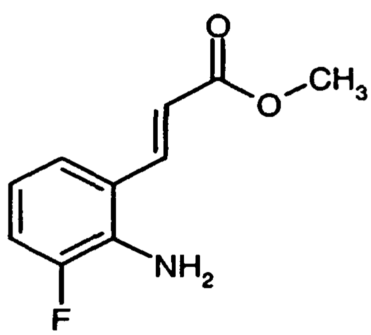

Ausgehend von 42.00 g (221.04 mmol) 2-Brom-6-fluoranilin werden nach der allgemeinen Arbeitsvorschrift [B] 29.66 g (68 % d. Th.) Produkt erhalten.
HPLC (Methode 1): $R_t$ = 4.14 min
MS (ESIpos): m/z = 196 $(M+H)^+$

**Beispiel 3A**

2-Amino-3-[(1E)-3-methoxy-3-oxo-1-propenyl]benzoesäuremethylester

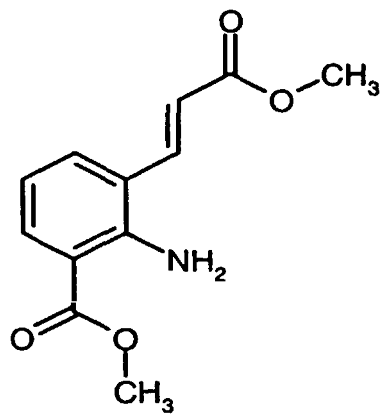

Ausgehend von 2.00 g (8.69 mmol) 2-Amino-3-brombenzoesäuremethylester werden nach der allgemeinen Arbeitsvorschrift [B] 1.29 g (60 % d. Th.) Produkt erhalten.
HPLC (Methode 1): $R_t$ = 4.42 min
MS (ESIpos): m/z = 236 $(M+H)^+$

**Beispiel 4A**

(2E)-3-[2-Amino-3,5-difluorphenyl]-2-propensäuremethylester

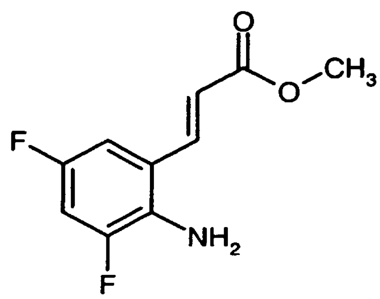

Ausgehend von 3.00 g (14.42 mmol) 2-Brom-4,6-difluoranilin werden nach der allgemeinen Arbeitsvorschrift [B] 1.41 g (45 % d. Th.) Produkt erhalten.
HPLC (Methode 1): $R_t$ = 4.23 min
MS (ESIpos): m/z = 214 $(M+H)^+$

**Beispiel 5A**

4-Amino-3-[(1E)-3-methoxy-3-oxo-1-propenyl]benzoesäuremethylester

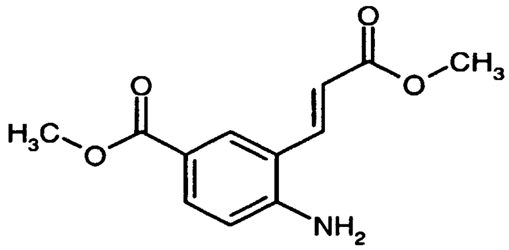

Ausgehend von 25.00 g (90.23 mmol) 4-Amino-3-iod-benzoesäuremethylester werden nach der allgemeinen Arbeitsvorschrift [B] 24.31 g (92 % d. Th.) Produkt erhalten.
HPLC (Methode 1): $R_t$ = 4.71 min
MS (ESIpos): m/z = 278 $(M+H)^+$

**Beispiel 6A**

(2E)-3-[2-Amino-5-cyanophenyl]-2-propensäuremethylester

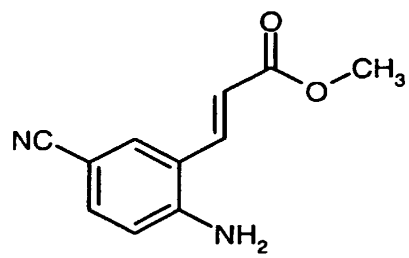

Ausgehend von 1.90 g (9.64 mmol) 3-Brom-4-aminobenzonitril werden nach der allgemeinen Arbeitsvorschrift [B] 1.28 g (50 % d. Th.) Produkt erhalten.
HPLC (Methode 1): $R_t$ = 2.85 min
MS (DCIpos): m/z = 220 $(M+NH_4)^+$

**Allgemeine Arbeitsvorschrift [C]: Synthese von substituierten 2-Nitrozimtsäurederivaten mittels Wittig-Horner-Reaktion aus 2-halogensubstituierten Benzaldehyden**

In einem 100 ml Einhalskolben werden 27.5 mmol Methyldiethylphosphonacetat, 25.0 mmol des Benzaldehyds mit 27.5 mmol Lithiumhydroxid in Tetrahydrofuran suspensiert. Nach beendeter Reaktion (Reaktionskontrolle mittels DC) wird der Ansatz mit gleichem Volumen Wasser versetzt. Man extrahiert die wässrige Phase dreimal mit Essigsäureethylester. Die vereinigten organischen Phasen werden dann mit gesättigter Natriumchlorid-Lösung gewaschen, über Natriumsulfat getrocknet und das Lösemittel entfernt. Das Produkt wird im Hochvakuum bei RT ohne weitere Reinigung getrocknet. Gegebenenfalls wird bei starker Verunreinigung säulenchromatographisch über Kieselgel mit Cyclohexan/Essigsäureethylester gereinigt.

**Beispiel 7A**

(2E)-3-(3-Methoxy-2-nitrophenyl)-2-propensäuremethylester

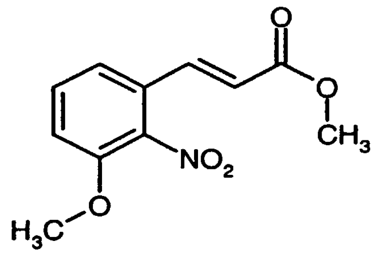

Ausgehend von 2.00 g (11.04 mmol) 3-Methoxy-2-nitrobenzaldehyd werden nach der allgemeinen Arbeitsvorschrift [C] 2.46 g (92 % d. Th.) Produkt erhalten.
HPLC (Methode 1): $R_t$= 4.37 min
MS (ESIpos): m/z = 238 $(M+H)^+$

**Beispiel 8A**

(2E)-3-(5-Fluor-2-nitrophenyl)-2-propensäuremethylester

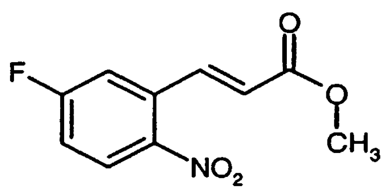

Ausgehend von 20.0 g (118.3 mmol) 5-Fluor-2-nitrobenzaldehyd werden nach der allgemeinen Arbeitsvorschrift [C] 7.25 g (27 % d. Th.) Produkt erhalten.
MS (DCI): m/z = 243 $(M+NH_4)^+$

**Allgemeine Arbeitsvorschrift [D]: Herstellung eines 2-Nitrobenzaldehyds aus einem Benzylhalogenid**

10.0 mmol des Benzylhalogenids werden mit 4.1 g Molekularsieb 4Å und 20.0 mmol *N*-Methylmorpholin-N-Oxid in 45 ml Acetonitril suspensiert. Man lässt bis zur Umsetzung (Reaktionskontrolle mittels DC) bei RT rühren. Nach beendeter Reaktion wird das Molekularsieb abfiltriert, das Lösungsmittel eingeengt und der Rückstand wieder in Essigsäureethylester aufgenommen. Diese Lösung wird zunächst mit 1N Salzsäure gewaschen und dann mit gesättigter Natriumchlorid-Lösung. Die abgetrennte organische Phase lässt man dann über Natriumsulfat trocknen und engt das Lösungsmittel wieder ein. Das Rohprodukt verfügt laut Analytik über eine genügend hohe Reinheit und kann direkt weiter umgesetzt werden.

**Beispiel 9A**

2-Fluor-6-nitrobenzaldehyd

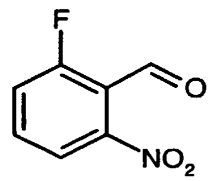

Ausgehend von 2.00 g (8.55 mmol) 3-Fluor-6-nitrobenzylbromid werden nach der allgemeinen Arbeitsvorschrift [D] 1.09 g (75 % d. Th.) Produkt erhalten.
HPLC (Methode 1): $R_t$ = 3.58 min

**Allgemeine Arbeitsvorschrift [E]: Reduktion der Nitrogruppe der 2-Nitrozimtsäurederivate**

In einem 250 ml Zweihalskolben werden unter Argon in 60 ml absolutem Ethanol 25 mmol der Nitroverbindung und 125 mmol Zinn-(II)-chloriddihydrat vorgelegt. Diese Suspension wird 30 Minuten unter Rückfluss gerührt, und es entsteht eine klare Lösung. Dann lässt man die Lösung auf Raumtemperatur abkühlen und gießt sie danach auf Eiswasser. Der pH-Wert wird entweder mit festem Natriumhydrogencarbonat oder mit einer gesättigten Natriumcarbonat-Lösung auf pH 7-8 eingestellt. Anschließend gibt man 60 ml Essigsäureethylester hinzu und filtriert die ausgefallenen Zinnsalze über ca. 1 cm Schichtdicke Kieselgur ab. Die organische Phase wird abgetrennt und die wässrige Phase noch einmal mit Essigsäureethylester extrahiert. Man vereinigt die organischen Phasen und wäscht sie einmal mit gesättigter Natriumchlorid-Lösung, trocknet sie über Natriumsulfat und engt das Lösemittel ca. um die Hälfte ein. Nun fügt man Aktivkohle hinzu, entsprechend 1% des Gewichts der Nitroverbindung, und erhitzt für 30 Minuten unter Rückfluss (Verfärbung der Lösung). Die Aktivkohle wird abfiltriert und das Lösemittel eingeengt. Der Rückstand wird im Hochvakuum getrocknet, und ohne weitere Aufreinigung erfolgt eine direkte Umsetzung zur nächsten Stufe.

**Beispiel 10A**

(2E)-3-[2-Aminophenyl]-propensäureinethylester

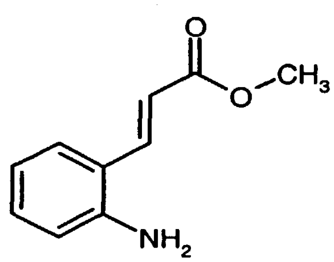

Ausgehend von 15.00 g (72.34 mmol) Nitroverbindung werden nach der allgemeinen Arbeitsvorschrift [E] 12.05 g (94 % d. Th.) Produkt erhalten.
LC-MS (Methode 6): $R_t$ = 3.29 min

**Beispiel 11A**

3-[2-Amino-6-fluorphenyl]-propensäuremethylester

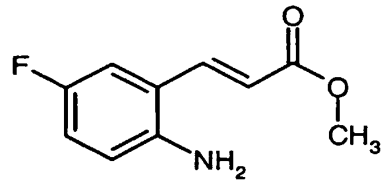

Ausgehend von 7.25 g (32.2 mmol) Nitroverbindung aus Beispiel 8A werden nach der allgemeinen Arbeitsvorschrift [E] 5.0 g (58 % d. Th.) Produkt erhalten.
HPLC (Methode 1): $R_t$= 3.33 min

**Allgemeine Arbeitsvorschrift [F]: Synthese der Iminophosphorane mittels Appel-Reaktion der substituierten Aniline**

In einem 50 ml Einhalskolben werden 10.0 mmol des 2-Aminozimtsäureesters, 20.0 mmol Triphenylphosphin, 100.0 mmol Tetrachlorkohlenstoff und 100.0 mmol Triethylamin in 20 ml Acetonitril gelöst. Man lässt 2 Stunden bei Raumtemperatur rühren. Nach beendeter Reaktion (Reaktionskontrolle mittels DC oder analytischer HPLC) wird das Lösungsmittel im Vakuum entfernt und der Rückstand durch Säulenchromatographie an Kieselgel mit Cyclohexan/Essigsäureethylester = 7:3 gereinigt.

**Beispiel 12A**

(2E)-3-{3-Fluor-2-[(triphenylphosphoranyliden)amino]phenyl}-propensäuremethylester

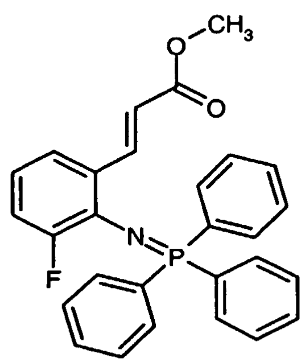

Ausgehend von 29.3 g (150.1 mmol) Aminverbindung aus Beispiel 2A werden nach der allgemeinen Arbeitsvorschrift [F] 55.0 g (80 % d. Th.) Produkt erhalten.
HPLC (Methode 1): $R_t$ = 4.46 min
MS (ESIpos): m/z = 456 $(M+H)^+$

**Beispiel 13A**

(2E)-3-{5-Fluor-2-[(triphenylphosphoranyliden)amino]phenyl}-propensäuremethylester

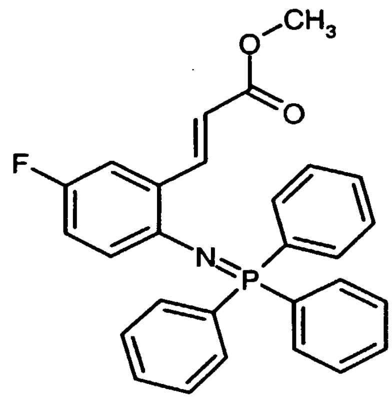

Ausgehend von 50.0 g (256.2 mmol) Aminverbindung aus Beispiel 11A werden nach der allgemeinen Arbeitsvorschrift [F] 89.6 g (77 % d. Th.) Produkt erhalten.
HPLC (Methode 1): $R_t$ = 4.36 min
MS (ESIpos): m/z = 456 $(M+H)^+$

**Beispiel 14A**

(2E)-3-{5-Cyano-2-[(triphenylphosphoranyliden)amino]phenyl}-propensäuremethylester

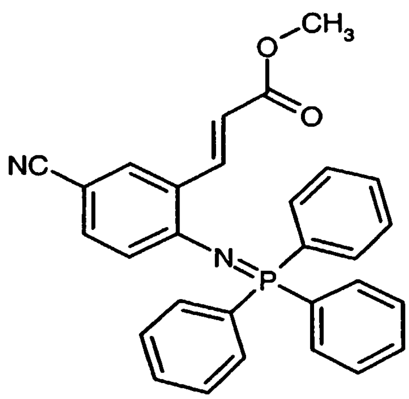

Ausgehend von 1.24 g (4.60 mmol) Aminverbindung aus Beispiel 6A werden nach der allgemeinen Arbeitsvorschrift [F] 2.12 g (92 % d. Th.) Produkt erhalten.
HPLC (Methode 1): $R_t$ = 4.42 min
MS (ESIpos): m/z = 463 $(M+H)^+$

**Allgemeine Arbeitsvorschrift [G]: Schutz von Indolen mit *tert*.-Butyloxycarbonyl**

7.6 mmol Indol und 9.2 mmol Di-*tert*.-butyldicarbonat werden in 16 ml Acetonitril vorgelegt und dann mit 0.8 mmol 4-Dimethylaminopyridin versetzt. Für 16 Stunden wird bei Raumtemperatur gerührt. Nach beendeter Reaktion (Reaktionskontrolle per DC) werden 25 ml Essigsäureethylester zur Reaktionslösung gegeben. Mit 50 ml Wasser und 50 ml einer gesättigten, wässrigen Natriumchlorid-Lösung wird die organische Phase gewaschen, anschließend über Natriumsulfat getrocknet und eingeengt.

**Beispiel 15A**

4-Methylindol-1-carbonsäure-*tert*.-butylester

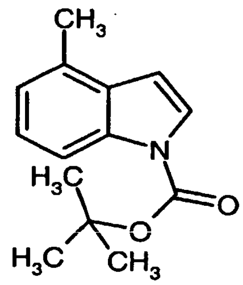

Ausgehend von 1.0 g (7.6 mmol) 4-Methylindol werden nach der allgemeinen Arbeitsvorschrift [G] 1.8 g (98 % d. Th.) Produkt erhalten.
HPLC (Methode 1): $R_t$ = 5.50 min
MS (ESIpos): m/z = 232 $(M+H)^+$

**Beispiel 16A**

4-Fluorindol-1-carbonsäure-*tert*.-butylester

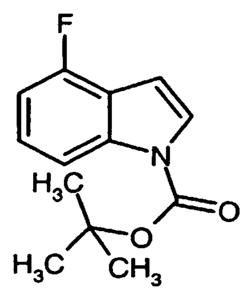

Ausgehend von 3.0 g (22.2 mmol) 4-Fluorindol werden nach der allgemeinen Arbeitsvorschrift [G] 5.1 g (97 % d. Th.) Produkt erhalten.
HPLC (Methode 1): $R_t$ = 5.53 min
MS (ESIpos): m/z = 236 $(M+H)^+$

**Beispiel 17A**

6-Methylindol-1-carbonsäure-*tert*.-butylester

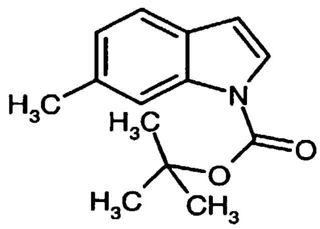

Ausgehend von 3.0 g (22.9 mmol) 6-Methylindol werden nach der allgemeinen Arbeitsvorschrift [G] 5.2 g (96 % d. Th.) Produkt erhalten.
HPLC (Methode 1): $R_t$ = 5.68 min
MS (ESIpos): m/z = 232 $(M+H)^+$

**Allgemeine Arbeitsvorschrift [H]: Darstellung von Indol-2-carbonsäureestern**

Unter Argon werden 7.8 mmol Indol-1-carbonsäure-*tert.*-butylester in 30 ml absolutem THF gelöst und auf -78˚C abgekühlt. 11.7 mmol *tert*.-Butyllithium (1.7M in Pentan) werden langsam addiert. Es wird 60 Minuten bei -78˚C gerührt, 23.3 mmol Chlorameisensäureethylester langsam addiert und zehn Minuten bei -78˚C gerührt. Anschließend wird über einen Zeitraum von 30 Minuten auf Raumtemperatur erwärmt und weitere 60 Minuten bei dieser Temperatur gerührt. Unter Eiskühlung werden 10 ml Wasser zugesetzt und mit 25 ml Essigsäureethylester extrahiert. Die wässrige Phase wird mit Essigsäureethylester extrahiert (zweimal 25 ml). Die vereinigten organischen Extrakte werden über Natriumsulfat getrocknet, eingeengt und säulenchromatographisch an Kieselgel (Laufmittel Cyclohexan/Essigsäureethylester = 25:1) gereinigt.

**Beispiel 18A**

4-Methylindol-1,2-dicarbonsäure-1-*tert*.-butylester-2-ethylester

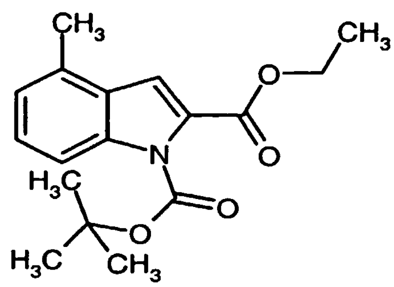

Ausgehend von 1.8 g (7.8 mmol) Boc-geschütztem 4-Methylindol (Beispiel 15A) werden nach der allgemeinen Arbeitsvorschrift [H] 1.5 g (62 % d. Th.) Produkt erhalten.
HPLC (Methode 1): $R_t$ = 5.30 min
MS (ESIpos): m/z = 304 $(M+H)^+$

**Beispiel 19A**

4-Fluorindol-1,2-dicarbonsäure-1-*tert*.-butylester-2-ethylester

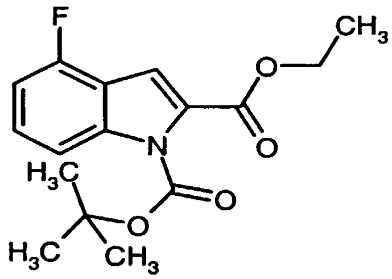

Ausgehend von 1.8 g (7.8 mmol) Boc-geschütztem 4-Fluorindol (Beispiel 16A) werden nach der allgemeinen Arbeitsvorschrift [H] 1.5 g (62 % d. Th.) Produkt erhalten.
HPLC (Methode 1): $R_t$ = 5.30 min
MS (ESIpos): m/z = 308 $(M+H)^+$

**Beispiel 20A**

6-Methylindol-1,2-dicarbonsäure-1-*tert.*-butylester-2-ethylester

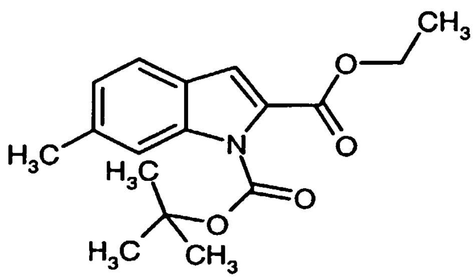

Ausgehend von 1.5 g (6.5 mmol) Boc-geschütztem 6-Methylindol (Beispiel 17A) werden nach der allgemeinen Arbeitsvorschrift [H] 1.0 g (48 % d. Th.) Produkt erhalten.
HPLC (Methode 1): $R_t$ = 5.60 min
MS (ESIpos): m/z = 290 $(M+H)^+$

**Allgemeine Arbeitsvorschrift [J]: Abspaltung der Boc-Schutzgruppe**

In einem 25 ml Einhalskolben werden 4.8 mmol des Boc-geschützten Indol Derivates vorgelegt und mit einem Dichlormethan/Trifluoressigsäure-Gemisch (4: 1) versetzt entsprechend 9.7 mmol Trifluoressigsäure. Man lässt 2 Stunden bei Raumtemperatur rühren. Nach beendeter Reaktion (Reaktionskontrolle per DC) wird das Lösungsmittel im Vakuum entfernt und das Produkt im Vakuum getrocknet.

**Beispiel 21A**

4-Methylindol-2-carbonsäureethylester

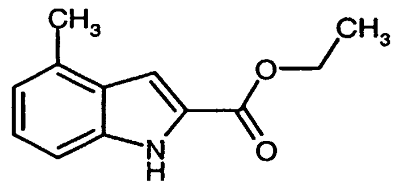

Ausgehend von 1.5 g (4.8 mmol) Boc-geschütztem Indolderivat aus Beispiel 18A werden nach der allgemeinen Arbeitsvorschrift [J] 0.9 g (94 % d. Th.) Produkt erhalten.
HPLC (Methode 1): $R_t$ = 4.70 min
MS (ESIpos): m/z = 204 $(M+H)^+$

**Beispiel 22A**

4-Fluorindol-2-carbonsäureethylester

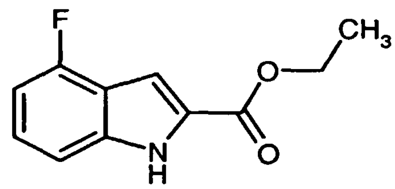

Ausgehend von 1.0 g (3.3 mmol) Boc-geschütztem Indolderivat aus Beispiel 19A werden nach der allgemeinen Arbeitsvorschrift [J] 0.6 g (88 % d. Th.) Produkt erhalten.
HPLC (Methode 1): $R_t$ = 4.69 min
MS (ESIpos): m/z = 208 $(M+H)^+$

**Beispiel 23A**

6-Methylindol-2-carbonsäureethylester

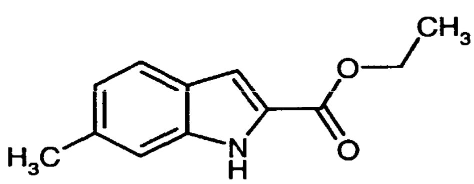

Ausgehend von 1.3 g (4.3 mmol) Boc-geschütztem Indolderivat aus Beispiel 20A werden nach der allgemeinen Arbeitsvorschrift [J] 0.8 g (96 % d. Th.) Produkt erhalten.
LC-MS (Methode 8): $R_t$ = 2.33 min
MS (ESIpos): m/z = 157 $(M -OCH_2CH_3 +H)^+$

**Allgemeine Arbeitsvorschrift [K]: Synthese der 1-Cyanomethylindole**

Unter Argon werden 4.6 mmol Indol-2-carbonsäureester in 10 ml N,N-Dimethylformamid gelöst und portionsweise mit 5.5 mmol Natriumhydrid (60 %ig in Mineralöl) versetzt. Nach der Zugabe rührt man bis zum Ende der Gasentwicklung (ca. 30 Minuten). Anschließend kühlt man auf 0°C ab und tropft 5.5 mmol Chloracetonitril zu. Es wird 16

Stunden bei Raumtemperatur gerührt. Der Ansatz wird mit Essigsäureethylester versetzt und mit Wasser und einer gesättigten wässrigen Natriumchlorid-Lösung gewaschen. Die organische Phase wird über Natriumsulfat getrocknet und eingeengt.

**Beispiel 24A**

1-Cyanomethyl-4-methyl-1H-indol-2-carbonsäureethylester

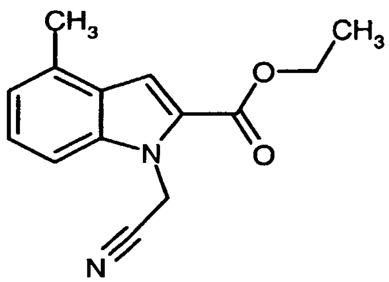

Ausgehend von 0.9 g (4.6 mmol) 4-Methylindol-2-carbonsäureethylester (Beispiel 21A) werden nach der allgemeinen Arbeitsvorschrift [K] 1.0 g (90 % d. Th.) Produkt erhalten.
HPLC (Methode 1): $R_t$ = 4.86 min
MS (ESIpos): m/z = 243 $(M+H)^+$

**Beispiel 25A**

1-Cyanomethyl-4-fluor-1H-indol-2-carbonsäureethylester

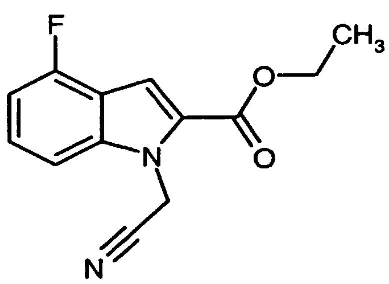

Ausgehend von 610 mg (2.9 mmol) 4-Fluorindol-2-carbonsäureethylester (Beispiel 22A) werden nach der allgemeinen Arbeitsvorschrift [K] 644 mg (89 % d. Th.) Produkt erhalten.
HPLC (Methode 1): $R_t$ = 4.86 min
MS (ESIpos): m/z = 247 $(M+H)^+$

**Beispiel 26A**

1-Cyanomethyl-6-methyl-1H-indol-2-carbonsäureethylester

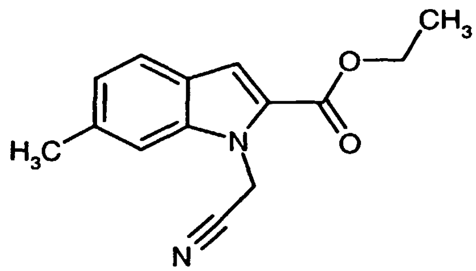

Ausgehend von 871 mg (4.3 mmol) 6-Methylindol-2-carbonsäureethylester (Beispiel 23A) werden nach der allgemeinen Arbeitsvorschrift [K] 1080 mg (88 % d. Th.) Produkt erhalten.
LC-MS (Methode 8): $R_t$ = 2.44 min
MS (ESIpos): m/z = 243 $(M+H)^+$

**Beispiel 27A**

1-Cyanomethyl-4-methoxy-1H-indol-2-carbonsäuremethylester

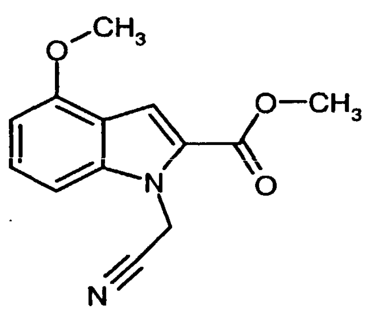

Ausgehend von 5.0 g (24.4 mmol) 4-Methoxyindol-2-carbonsäuremethylester werden nach der allgemeinen Arbeitsvorschrift [K] 5.6 g (92 % d. Th.) Produkt erhalten.
HPLC (Methode 1): $R_t$= 4.34 min
MS (ESIpos): m/z = 245 $(M+H)^+$

**Beispiel 28A**

1-Cyanomethyl-6-methoxy-1H-indol-2-carbonsäuremethylester

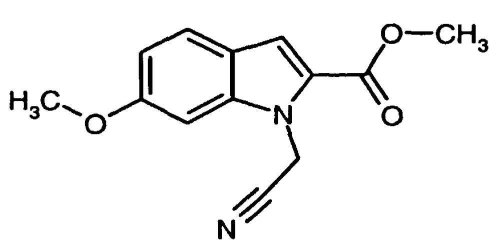

Ausgehend von 500 mg (2.4 mmol) 6-Methoxyindol-2-carbonsäuremethylester werden nach der allgemeinen Arbeitsvorschrift [K] 460 mg (75 % d. Th.) Produkt erhalten.

HPLC (Methode 1): $R_t$ = 4.42 min
MS (ESIpos): m/z = 245 $(M+H)^+$

**Beispiel 29A**

1-Cyanomethyl-5-fluor-1H-indol-2-carbonsäureethylester

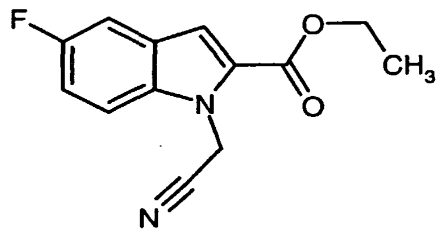

Ausgehend von 5.2 g (24.9 mmol) 5-Fluorindol-2-carbonsäureethylester werden nach der allgemeinen Arbeitsvorschrift [K] 5.5 g (88 % d. Th.) Produkt erhalten.
HPLC (Methode 1): $R_t$= 4.62 min
MS (ESIpos): m/z = 247 $(M+H)^+$

**Beispiel 30A**

1-Cyanomethyl-5-methyl-1H-indol-2-carbonsäuremethylester

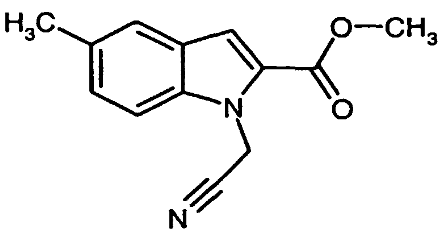

Ausgehend von 250 mg (1.3 mmol) 5-Methylindol-2-carbonsäuremethylester werden nach der allgemeinen Arbeitsvorschrift [K] 200 mg (66 % d. Th.) Produkt erhalten.
HPLC (Methode 1): $R_t$= 4.61 min
MS (ESIpos): m/z = 229 $(M+H)^+$

**Allgemeine Arbeitsvorschrift [L]: Reduktion der Cyanomethylindole zu Pyrazino[1,2-a]indolen**

Zu einer Suspension von 53.7 mmol Lithiumaluminiumhydrid in 100 ml Diethylether wird unter Argon langsam eine Suspension von 22.4 mmol 1-Cyanomethylindol-2-carbonsäureester in 170 ml Diethylether gegeben und anschließend vier Stunden unter Rückfluss gerührt. Nach beendeter Reaktion (Reaktionskontrolle per HPLC) wird der Ansatz vorsichtig mit 30 ml einer gesättigten wässrigen Ammoniumchlorid-Lösung versetzt. Es werden zweimal je 200 ml Essigsäureethylester zugegeben und zehn Minuten bei 80°C gerührt. Das Reaktionsgemisch wird filtriert, die organische Phase mit einer gesättigten wässrigen Ammoniumchlorid-Lösung gewaschen (einmal 200 ml), über Natriumsulfat getrocknet, eingeengt und an Kieselgel (Dichlormethan/ Methanol = 50:1) säulenchromatographisch gereinigt.

**Beispiel 31A**

9-Methyl-1,2,3,4-tetrahydro-pyrazino[1,2-a]indol

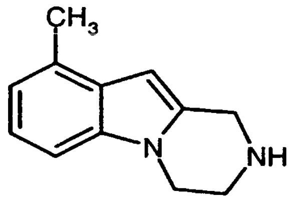

Ausgehend von 1.1 g (4.3 mmol) 1-Cyano-4-methylindol-2-carbonsäureethylester (Beispiel 24A) werden nach der allgemeinen Arbeitsvorschrift [L] 235 mg (29 % d. Th.) Produkt erhalten.
HPLC (Methode 1): $R_t$= 3.68 min
MS (ESIpos): m/z = 187 $(M+H)^+$

**Beispiel 32A**

9-Fluor-1,2,3,4-tetrahydro-pyrazino[1,2-a]indol

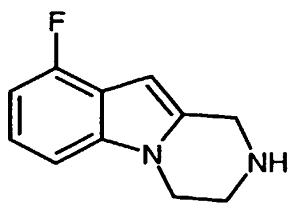

Ausgehend von 632 g (2.6 mmol) 1-Cyano-4-fluorindol-2-carbonsäureethylester (Beispiel 25A) werden nach der allgemeinen Arbeitsvorschrift [L] 90 mg (18 % d. Th.) Produkt erhalten.
LC-MS (Methode 5): $R_t$= 1.67 min
MS (ESIpos): m/z = 191 $(M+H)^+$

**Beispiel 33A**

7-Methyl-1,2,3,4-tetrahydro-pyrazino[1,2-a]indol

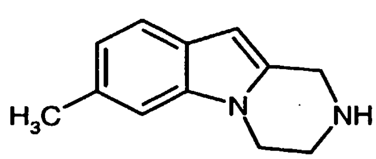

Ausgehend von 1.08 g (3.8 mmol) 1-Cyano-6-methylindol-2-carbonsäuremethylester (Beispiel 26A) werden nach der allgemeinen Arbeitsvorschrift [L] 430 mg (61 % d. Th.) Produkt erhalten.
LC-MS (Methode 3): $R_t$ = 1.62 min

MS (ESIpos): m/z = 187 $(M+H)^+$

**Beispiel 34A**

9-Methoxy-1,2,3,4-tetrahydro-pyrazino[1,2-a]indol

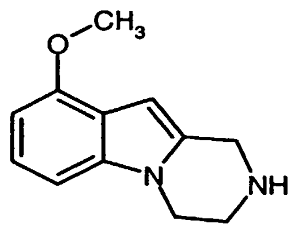

Ausgehend von 433 mg (1.7 mmol) 1-Cyano-4-methoxyindol-2-carbonsäuremethylester (Beispiel 27A) werden nach der allgemeinen Arbeitsvorschrift [L] 143 mg (41 % d. Th.) Produkt erhalten.
HPLC (Methode 1): $R_t$ = 3.46 min
MS (ESIpos): m/z = 203 $(M+H)^+$

**Beispiel 35A**

7-Methoxy-1,2,3,4-tetrahydro-pyrazino[1,2-a]indol

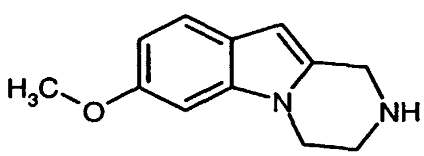

Ausgehend von 430 mg (1.8 mmol) 1-Cyano-6-methoxyindol-2-Carbonsäuremethylester (Beispiel 28A) werden nach der allgemeinen Arbeitsvorschrift [L] 172 mg (24 % d. Th.) Produkt erhalten.
HPLC (Methode 1): $R_t$ = 3.49 min
MS (ESIpos): m/z = 203 $(M+H)^+$

**Beispiel 36A**

8-Fluor-1,2,3,4-tetrahydro-pyrazino[1,2-a]indol

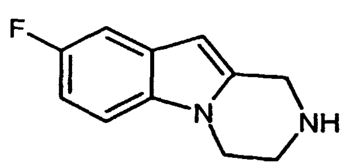

Ausgehend von 5.5 g (22.4 mmol) 1-Cyano-5-fluorindol-2-carbonsäureethylester (Beispiel 29A) werden nach der allgemeinen Arbeitsvorschrift [L] 1.8 g (43 % d. Th.) Produkt erhalten.
HPLC (Methode 1): $R_t$ = 3.52 min
MS (ESIpos): m/z = 191 $(M+H)^+$

**Beispiel 37A**

8-Methyl-1,2,3,4-tetrahydro-pyrazino[1,2-a]indol

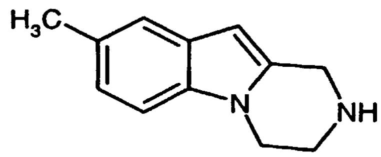

Ausgehend von 170 mg (0.7 mmol) 1-Cyano-5-methylindol-2-Carbonsäuremethylester (Beispiel 30A) werden nach der allgemeinen Arbeitsvorschrift [L] 88 mg (59 % d. Th.) Produkt erhalten.
LC-MS (Methode 7): $R_t$ = 2.38 min
MS (ESIpos): m/z = 187 $(M+H)^+$

**Allgemeine Arbeitsvorschrift [M]: Umsetzung eines Iminophosphorans mit einem Isocyanat und anschließende Umsetzung zum Dihydrochinazolin-Derivat mit einem Amin**

1.0 Äquivalente des Iminophosphorans werden in 20 ml Dichlormethan gelöst (0.1-0.2M Lösung). Danach werden 1.0 Äquivalente eines substituierten Isocyanats hinzugefügt und man lässt bis zur Beendigung der Reaktion bei RT rühren. Eine Reaktionskontrolle erfolgt durch DC oder analytische HPLC.
Die so erhaltene Lösung des Carbodiimids in Dichlormethan wird mit 1.0 Äquivalenten Amin sowie einer Spatelspitze Kieselgel versetzt und bei Raumtemperatur bis zur vollständigen Umsetzung gerührt. Nach beendeter Reaktion (Reaktionskontrolle mittels DC oder HPLC) wird der Ansatz eingeengt und durch präparative HPLC an RP-Phase gereinigt.
Unter Umständen zeigt das NMR noch einen schwankenden Anteil an nicht cyclisiertem Reaktionsprodukt an. In diesen Fällen wird das Gemisch aus cyclisiertem und nicht cyclisiertem Produkt in Dioxan aufgenommen, mit einer Spatelspitze Kieselgel versetzt und unter Rückfluss 30 min bis 16 h gerührt. Das Kieselgel wird abfiltriert und die Lösung für weitere Umsetzungen verwendet.

**Beispiel 38A**

[8-Fluor-3-(2-methoxy-5-trifluormethyl-phenyl)-2-(9-methyl-3,4-dihydro-1H-pyrazino[1,2-a]-indol-2-yl)-3,4-dihydro-chinazolin-4-yl]essigsäuremethylester

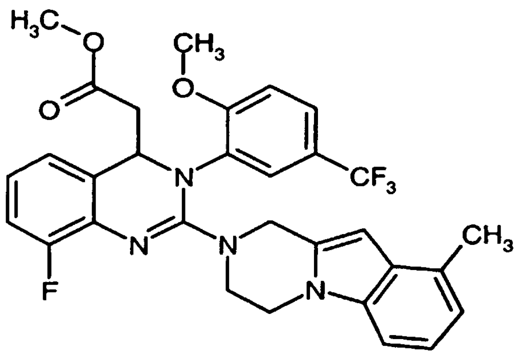

Ausgehend von 563 mg (1.2 mmol) Iminophosphoran aus Beispiel 12A, 268 mg (1.2 mmol) 2-Iso-cyanato-1-methoxy-4-(trifluormethyl)benzol und 230 mg (1.2 mmol) 9-Methyl-1,2,3,4-tetrahydropyrazino[1,2-a]indol aus Beispiel 31A werden nach der allgemeinen Arbeitsvorschrift [M] 325 mg (42 % d. Th.) Produkt erhalten.
HPLC (Methode 1): $R_t$=4.98 min
MS (ESIpos): m/z = 581 $(M+H)^+$

**Beispiel 39A**

[8-Fluor-3-(2-methoxy-5-trifluormethyl-phenyl)-2-(9-fluor-3,4-dihydro-1H-pyrazino[1,2-a]-indol-2-yl)-3,4-dihydro-chinazolin-4-yl]essigsäuremethylester

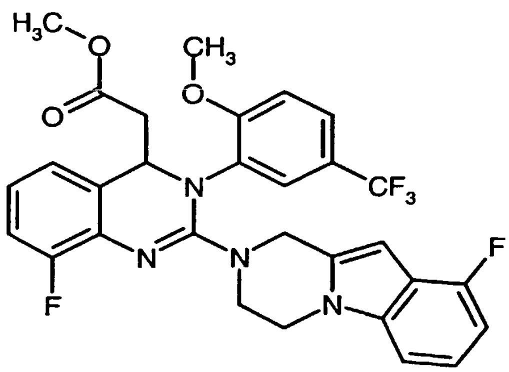

Ausgehend von 192 mg (0.4 mmol) Iminophosphoran aus Beispiel 12A, 91 mg (0.4 mmol) 2-Isocyanato-1-methoxy-4-(trifluormethyl)benzol und 80 mg (0.4 mmol) 9-Fluor-1,2,3,4-tetrahydropyrazino[1,2-a]indol aus Beispiel 32A werden nach der allgemeinen Arbeitsvorschrift [M] 78 mg (30 % d. Th.) Produkt erhalten.
HPLC (Methode 1): $R_t$ = 4.98 min
MS (ESIpos): m/z = 585 $(M+H)^+$

**Beispiel 40A**

[8-Fluor-3-(2-methoxy-5-trifluormethyl-phenyl)-2-(7-methyl-3,4-dihydro-1H-pyrazino[1,2-a]-indol-2-yl)-3,4-dihydro-chinazolin-4-yl]essigsäuremethylester

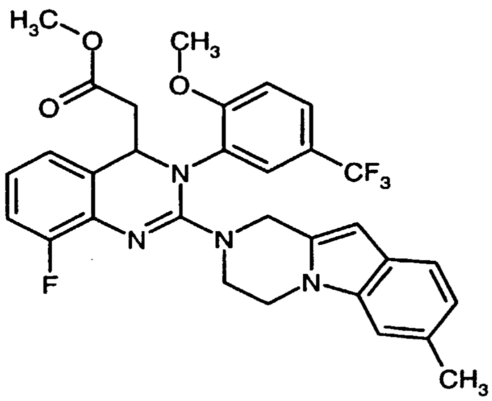

Ausgehend von 244 mg (0.5 mmol) Iminophosphoran aus Beispiel 12A, 105 mg (0.5 mmol) 2-Isocyanato-1-methoxy-4-(trifluormethyl)benzol und 100 mg (0.5 mmol) 9-Methyl-1,2,3,4-tetrahydropyrazino[1,2-a]indol aus Beispiel 33A werden nach der allgemeinen Arbeitsvorschrift [M] 1 mg (1 % d. Th.) Produkt erhalten.
LC-MS (Methode 3): $R_t$ = 3.51 min
MS (ESIpos): m/z = 551 $(M+H)^+$

## Beispiel 41A

[8-Fluor-3-(2-methoxy-5-trifluormethyl-phenyl)-2-(9-methoxy-3,4-dihydro-1 H-pyrazino[1,2-a]-indol-2-yl)-3,4-dihydro-chinazolin-4-yl]essigsäuremethytester

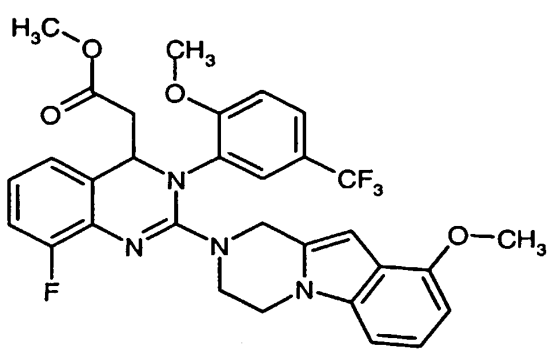

Ausgehend von 500 mg (1.1 mmol) Iminophosphoran aus Beispiel 12A, 238 mg (1.1 mmol) 2-Isocyanato-1-methoxy-4-(trifluormethyl)benzol und 222 mg (1.1 mmol) 9-Methoxy-1,2,3,4-tetrahydro-pyrazino[1,2-a]indol aus Beispiel 34A werden nach der allgemeinen Arbeitsvorschrift [M] 270 mg (38 % d. Th.) Produkt erhalten.
HPLC (Methode 1): $R_t$ = 4.73 min
MS (ESIpos): m/z = 597 $(M+H)^+$

## Beispiel 42A

{8-Fluor-2-(9-methoxy-3,4-dihydropyrazino[1,2-a]indol-2-yl)-3-[3-(trifluormethyl)-phenyl]-3,4-dihydro-chinazolin-4-yl} essigsäuremethylester

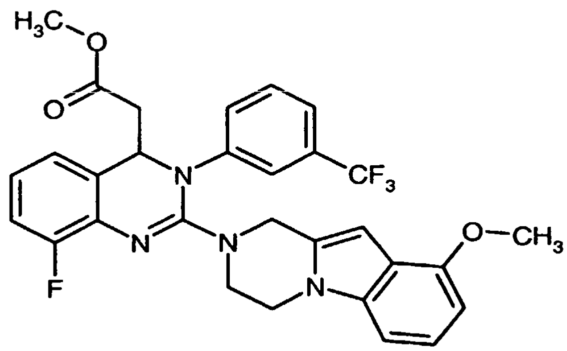

Ausgehend von 281 mg (0.6 mmol) Iminophosphoran aus Beispiel 12A, 134 mg (0.6 mmol) 1-Isocyanato-3-(trifluormethyl)benzol und 129 mg (0.6 mmol) 9-Methoxy-1,2,3,4-tetrahydro-pyrazino-[1,2-a]indol aus Beispiel 34A werden nach der allgemeinen Arbeitsvorschrift [M] 239 mg (66 % d. Th.) Produkt erhalten.
HPLC (Methode 1): $R_t$ = 4.76 min
MS (ESIpos): m/z = 567 $(M+H)^+$

**Beispiel 43A**

{8-Fluor-2-(7-methoxy-3,4-dihydropyrazino[1,2-a]indol-2-yl)-3-[3-(trifluormethyl)-phenyl]-3,4-dihydro-chinazolin-4-yl} essigsäuremethylester

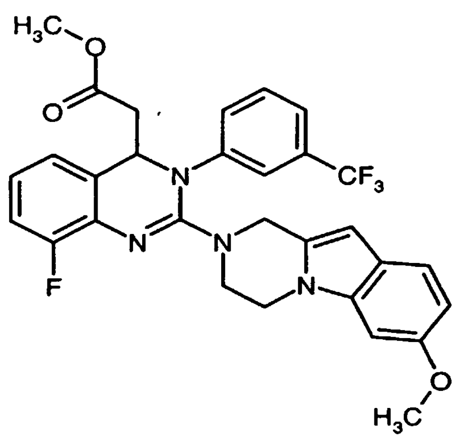

Ausgehend von 182 mg (0.4 mmol) Iminophosphoran aus Beispiel 12A, 134 mg (0.4 mmol) 1-Isocyanato-3-(trifluormethyl)benzol und 81 mg (0.4 mmol) 7-Methoxy-1,2,3,4-tetrahydro-pyrazino[1,2-a]indol aus Beispiel 35A werden nach der allgemeinen Arbeitsvorschrift [M] 140 mg (62 % d. Th.) Produkt erhalten.
HPLC (Methode 1): $R_t$ = 4.72 min
MS (ESIpos): m/z = 567 $(M+H)^+$

**Beispiel 44A**

[8-Fluor-3-(2-methoxy-5-trifluormethyl-phenyl)-2-(8-fluor-3,4-dihydro-1H-pyrazino[1,2-a]-indol-2-yl)-3,4-dihydro-chinazolin-4-yl]essigsäuremethylester

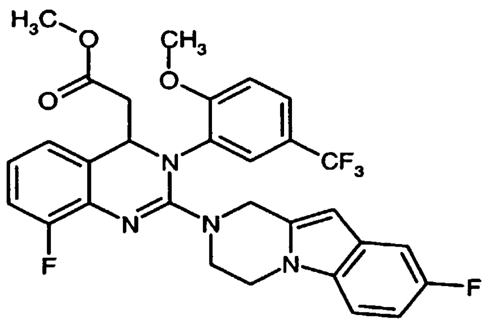

Ausgehend von 182 mg (0.4 mmol) Iminophosphoran aus Beispiel 12A, 86 mg (0.4 mmol) 2-Isocyanato-1-methoxy-4-(trifluormethyl)benzol und 130 mg (0.4 mmol) 8-Fluor-1,2,3,4-tetrahydropyrazino[1,2-a]indol aus Beispiel 36A werden nach der allgemeinen Arbeitsvorschrift [M] 95 g (35 % d. Th.) Produkt erhalten.
HPLC (Methode 1): $R_t$ = 4.80 min
MS (ESIpos): m/z = 585 $(M+H)^+$

**Beispiel 45A**

{8-Fluor-2-(8-fluor-3,4-dihydropyrazino[1,2-a]indol-2-yl)-3-[3-(trifluormethyl)-phenyl]-3,4-dihydro-chinazolin-4-yl}essigsäuremethylester

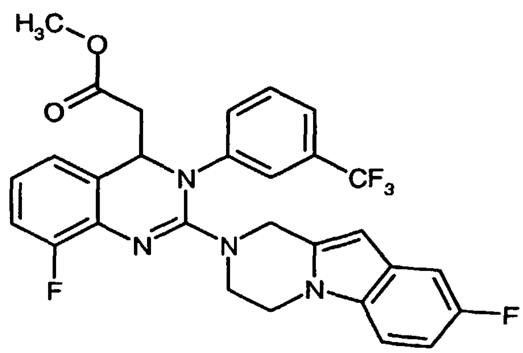

Ausgehend von 546 mg (1.2 mmol) Iminophosphoran aus Beispiel 12A, 402 mg (1.2 mmol) 1-Isocyanato-3-(trifluormethyl)benzol und 240 mg (1.3 mmol) 8-Fluor-1,2,3,4-tetrahydro-pyrazino[1,2-a]indol aus Beispiel 36A werden nach der allgemeinen Arbeitsvorschrift [M] 610 g (86 % d. Th.) Produkt erhalten.
HPLC (Methode 1): $R_t$= 3.96 min
MS (ESIpos): m/z = 555 $(M+H)^+$

**Beispiel 46A**

{8-Fluor-2-(8-methyl-3,4-dihydropyrazino[1,2-a]indol-2-yl)-3-[3-(trifluormethyl)-phenyl]-3,4-dihydro-chinazolin-4-yl}essigsäuremethylester

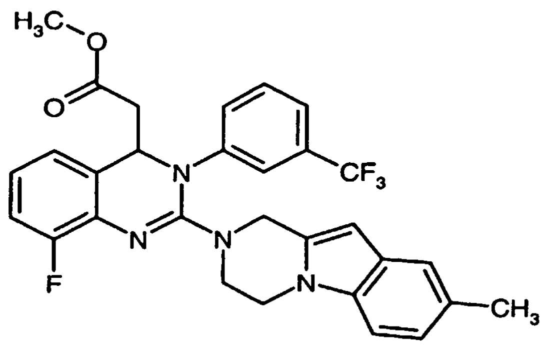

Ausgehend von 182 mg (0.4 mmol) Iminophosphoran aus Beispiel 12A, 134mg (0.4 mmol) 1-Isocyanato-3-(trifluormethyl)benzol und 80 mg (0.4 mmol) 8-Methyl-1,2,3,4-tetrahydro-pyrazino[1,2-a]indol aus Beispiel 37A werden nach der allgemeinen Arbeitsvorschrift [M] 28 mg (12 % d. Th.) Produkt erhalten.
LC-MS (Methode 3): $R_t$= 3.90 min
MS (ESIpos): m/z = 551 $(M+H)^+$

**Allgemeine Arbeitsvorschrift [N]: Darstellung von substituierten 2-Oxo-3-(2-nitrophenyl)-propansäureethylestern**

16.26 mmol Kalium-*tert*.-butylat werden in 40 ml wasserfreiem Diethylether und 4 ml absolutem Ethanol gelöst, mit 16.26 mmol Oxalsäurediethylester versetzt und 5 Minuten bei RT gerührt. Anschließend werden 14.78 mmol des entsprechenden 2-Oxo-3-(2-nitrophenyl)-propansäure-ethylesters zugegeben und 1 Stunde bei RT gerührt. Die entstandene Suspension wird mit gesättigter Ammoniumchlorid-Lösung und Eisessig versetzt, die organische Phase mit Wasser, gesättigter Natriumhydrogencarbonat-Lösung und gesättigter Natriumchlorid-Lösung gewaschen, über Natriumsulfat getrocknet und eingeengt.

**Beispiel 47A**

2-Oxo-3-(3-fluor-2-methyl-6-nitrophenyl)-propansäureethylester

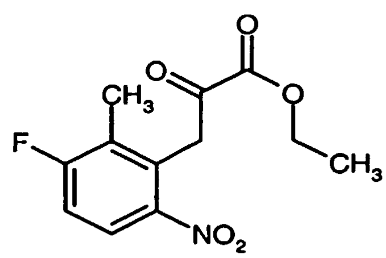

Ausgehend von 2.50 g (14.78 mmol) 2,3-Dimethyl-4-fluornitrobenzol werden nach der allgemeinen Arbeitsvorschrift [N] 2.43 g (57 % d. Th.) Produkt erhalten.
HPLC (Methode 1): $R_t$ = 4.51 min
MS (DCI): m/z = 287 $(M+NH_4)^+$

**Arbeitsvorschrift [O]: Darstellung von substituierten Indol-2-carbonsäureestern**

Ein Gemisch aus 8.36 mmol des entsprechenden 2-Oxo-3-(2-nitrophenyl)-propansäureethylesters und 75.24 mmol Eisenpulver in 15 ml Ethanol und 15 ml Essigsäure wird 2 Stunden unter Rückfluss erhitzt. Anschließend wird die Suspension eingeengt, der Rückstand dreimal mit Essigsäureethylester aufgeschlämmt, über Kieselgur filtriert und

eingeengt.

**Beispiel 48A**

5-Fluor-4-methylindol-2-carbonsäureethylester

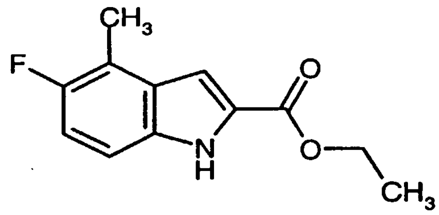

Ausgehend von 2.42 g (8.36 mmol) 2-Oxo-3-(3-fluor-2-methyl-6-nitrophenyl)-propansäure-ethylester werden nach der allgemeinen Arbeitsvorschrift [O] 1.49 g (81 % d. Th.) Produkt erhalten.
HPLC (Methode 1): $R_t$= 4.79 min
MS (DCI): m/z = 222 $(M+H)^+$

**Beispiel 49A**

8-Fluor-1,2,3,4,10,10a-hexahydropyrazino[1,2-a]indol

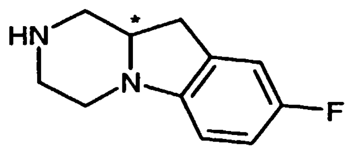

1.37g (7.20 mmol) Indolpiperazin aus Beispiel 36A werden in 28 ml Eisessig gelöst und portionsweise mit 1.81g (28.81 mmol) Natriumcyanoborhydrid versetzt. Es wird 16 Stunden bei RT gerührt, 30 ml Wasser zugesetzt und portionsweise in 200 ml eisgekühlte 10 %ige wässrige Natronlauge-Lösung eingerührt. Die wässrige Phase wird zweimal mit je 50 ml Dichlormethan extrahiert. Die vereinigten organischen Extrakte werden über Natriumsulfat getrocknet und eingeengt. Es werden 1.07 g (77 % d. Th.) Produkt erhalten.

Enantiomerentrennung nach Methode 9 ergibt 510 mg des Enantiomers.
HPLC (Methode 1): $R_t$ = 3.43 min
MS (ESIpos): m/z = 193 $(M+H)^+$

**Beispiel 50A**

{8-Fluor-2-(8-fluor-3,4,10,10a-tetrahydropyrazino[1,2-a]indol-2(1H)-yl)-3-[2-methoxy-5-(trifluormethyl)phenyl]-3,4-dihydro-quinazolin-4-yl}essigsäuremethylester

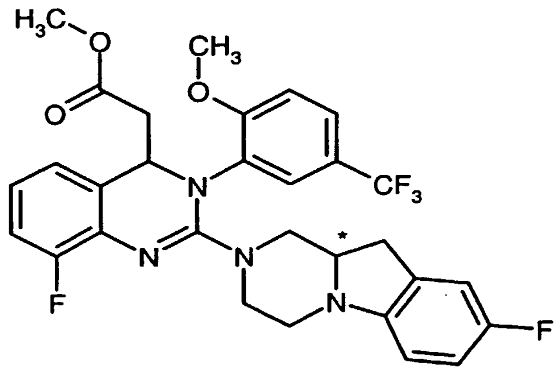

Ausgehend von 323 mg (0.7 mmol) Iminophophoran aus Beispiel 12A, 154 mg (0.7 mmol) 1-Isocyanato-3-(trifluormethyl)benzol und 150 mg (0.7 mmol) 8-Fluor-1,2,3,4,10,10a-hexahydro-pyrazino[1,2-a]indol aus Beispiel 49A werden nach der allgemeinen Arbeitsvorschrift [M] 235 mg (55 % d. Th.) Produkt erhalten.
HPLC (Methode 1): $R_t$ = 4.88 min
MS (ESIpos): m/z = 587(M+H)$^+$

**Ausführungsbeispiele**

**Allgemeine Arbeitsvorschrift [P]: Esterverseifung der Chinazolinylessigsäurester**

Es werden 1.0 Äquivalente des Chinazolinylessigsäuresters in Dioxan gelöst und 3.0 Äquivalente 1N Natronlauge hinzugefügt. Man lässt für 16 Stunden bei 50°C rühren und nach beendeter Reaktion wird der Ansatz eingeengt. Der Rückstand wird dann in Wasser aufgenommen und mit 1N Salzsäure auf pH 5 gestellt. Man filtriert den entstehenden Niederschlag ab, wäscht ihn mit wenig Wasser und Diethylether und trocknet ihn im Hochvakuum bei Raumtemperatur. Falls die Reinheit des Produktes nicht hoch genug ist, wird es über präparative HPLC an RP-Phase gereinigt (Methode 2).

**Beispiel 1**

{8-Fluor-2-(9-methyl-3,4-dihydropyrazino[1,2-a]indol-2(1H)-yl)-3-[3-(trifluormethyl)phenyl]-3,4-dihydro-chinazolin-4-yl } essigsäure

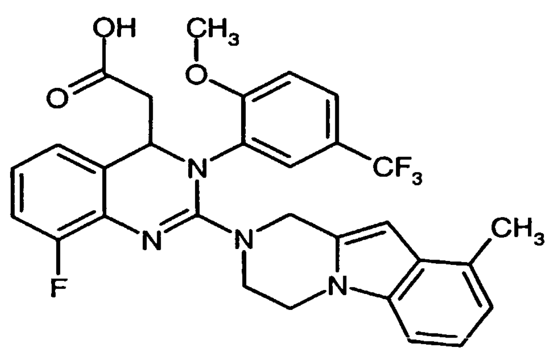

Ausgehend von 315 mg (0.5 mmol) Methylester aus Beispiel 38A werden nach der allgemeinen Arbeitsvorschrift [P] 330 mg (99 % d. Th.) Produkt erhalten.
HPLC (Methode 1): $R_t$= 4.75 min
MS (ESIpos): m/z = 567 (M+H)$^+$
$^1$H-NMR (400 MHZ, $CD_3CN$): δ [ppm] = 7.51 (d, 1H); 7.16-7.02 (m, 3H); 6.97-6.78 (m, 4H); 6.25 (s, 1H); 4.89 (dd, 1H); 4.75 (dd, 1H); 4.00-3.93 (m, 1H); 3.82-3.75 (m, 2H); 3.65 (brs, 3H); 3.53-3.47 (m, 4H); 2.78 (dd, 1H); 2.40 (s, 3H).

**Beispiel 2**

{8-Fluor-2-(9-methyl-3,4-dihydropyrazino[1,2-a]indol-2(1H)-yl)-3-[3-(trifluormethyl)phenyl]-3,4-dihydro-chinazolin-4-yl} essigsäure

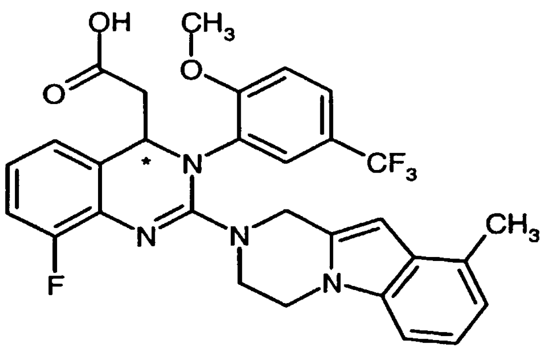

Ausgehend von 330 mg der Chinazolinylessigsäure aus Beispiel 1 werden nach Enantiomerentrennung (Methode 4) 90 mg des Enantiomers erhalten.
$^1$H-NMR (400 MHZ, $CD_3CN$): δ [ppm] = 7.45 (d, 1H); 7.07-6.81 (m, 7H); 6.25 (s, 1H); 4.91 (dd, 1H); 4.73 (dd, 1H); 3.91-3.88 (m, 4H); 3.78-3.72 (brs, 3H); 3.54-3.48 (m, 2H); 2.87 (dd, 1H); 2.50 (dd, 1H); 2.43 (s, 3H).

**Beispiel 3**

8-Fluor-2-(9-fluor-3,4-dihydropyrazino[1,2-a]indol-2(1H)-yl)-3-[3-(trifluormethyl)phenyl]-3,4-dihydro-chinazolin-4-yl}essigsäure

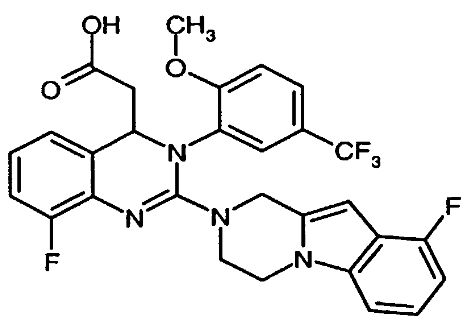

Ausgehend von 65 mg (0.1 mmol) des Methylesters aus Beispiel 39A werden nach der allgemeinen Arbeitsvorschrift [P] 62 mg (90 % d. Th.) Produkt erhalten.
HPLC (Methode 1): $R_t$ = 4.68 min
MS (ESIpos): m/z = 571 $(M+H)^+$
$^1$H-NMR (400 MHZ, DMSO-$d_6$): δ [ppm] = 7.51 (d, 1H); 7.21-6.75 (m, 7H); 6.27 (s, 1H); 4.95-4.89 (m, 1H); 4.75 (dd, 1H); 4.00 (dd, 2H); 3.82-3.78 (m, 2H); 3.69-3.63 (m, 2H); 3.56 (brs, 3H), 2.83-2.69 (m, 1H); 2.50-2.42 (m, 1H).

**Beispiel 4**

8-Fluor-2-(9-fluor-3,4-dihydropyrazino[1,2-a]indol-2(1H)-yl)-3-[3-(trifluormethyl)phenyl]-3,4-dihydro-chinazolin-4-yl}essigsäure

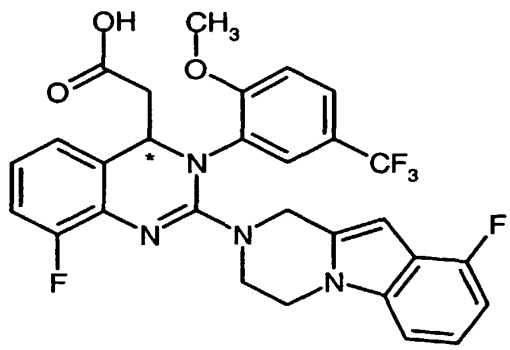

Ausgehend von 62 mg der Chinazolinylessigsäure aus Beispiel 3 werden nach Enantiomerentrennung (Methode 4) 27 mg des Enantiomers erhalten.
$^1$H-NMR (400 MHZ, $CD_3CN$): δ [ppm] = 7.53 (d, 1H); 7.15-6.72 (m, 7H); 6.23 (s, 1H); 4.90 (dd, 1H); 4.73 (dd, 1H); 3.99-3.94 (m, 2H); 3.80-3.73 (m, 5H); 3.61-3.56 (m, 2H); 2.88 (dd, 1H); 2.52 (dd, 1H).

**Beispiel 5**

{8-Fluor-2-(9-methoxy-3,4-dihydropyrazino[1,2-a]indol-2(1H)-yl)-3-[3-(trifluormethyl)phenyl]-3,4-dihydro-chinazolin-4-yl}essigsäure

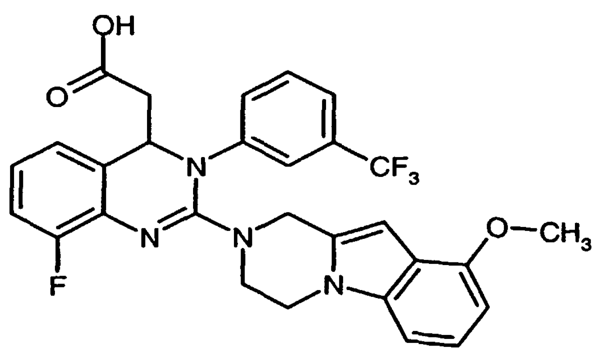

Ausgehend von 228 mg (0.4 mmol) des Methylesters aus Beispiel 42A werden nach der allgemeinen Arbeitsvorschrift [P] 228 mg (99 % d. Th.) Produkt erhalten.
HPLC (Methode 1): $R_t$= 4.55 min
MS (ESIpos): m/z = 553 $(M+H)^+$

**Beispiel 6**

{8-Fluor-2-(8-fluor-3,4-dihydropyrazino[1,2-a]indol-2(1H)-yl)-3-[3-(trifluormethyl)phenyl]-3,4-dihydro-chinazolin-4-yl} essigsäure

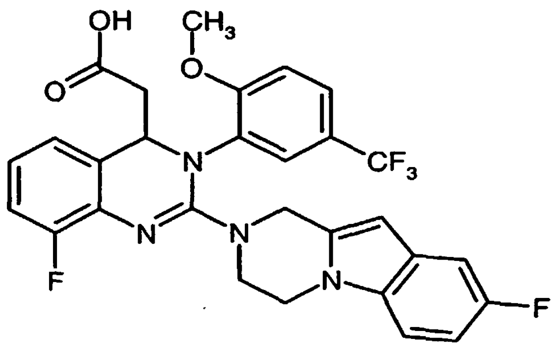

Ausgehend von 80 mg (0.1 mmol) des Methylesters aus Beispiel 44A werden nach der allgemeinen Arbeitsvorschrift [P] 74 mg (96 % d. Th.) Produkt erhalten.
HPLC (Methode 1): $R_t$ = 4.65 min
MS (ESIpos): m/z = 571 $(M+H)^+$
$^1$H-NMR (400 MHZ, $CD_3CN$): δ [ppm] = 7.51 (d, 1H); 7.21-6.86 (m, 7H); 6.14 (s, 1H); 5.10-4.95 (m, 1H); 4.77 (dd, 1H), 4.02-3.96 (m, 4H); 3.65-3.59 (m, 5H); 3.03 (dd, 1H); 2.66 (dd, 1H).

**Beispiel 7**

{8-Fluor-2-(8-fluor-3,4-dihydropyrazino[1,2-a]indol-2(1H)-yl)-3-[3-(trifluormethyl)phenyl]-3,4-dihydro-chinazolin-4-yl} essigsäure

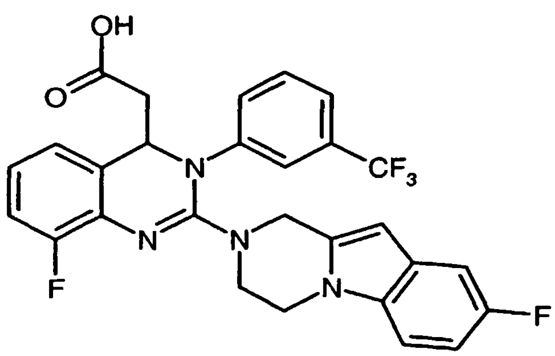

Ausgehend von 130 mg (0.2 mmol) des Methylesters aus Beispiel 45A werden nach der allgemeinen Arbeitsvorschrift [P] 120 mg (93 % d. Th.) Produkt erhalten.
HPLC (Methode 1): $R_t$= 4.63 min
MS (ESIpos): m/z = 541 $(M+H)^+$

**Beispiel 8**

{8-Fluor-2-(8-fluor-3,4-dihydropyrazino[1,2-a]indol-2(1H)-yl)-3-[3-(trifluormethyl)phenyl]-3,4-dihydro-chinazolin-4-yl} essigsäure

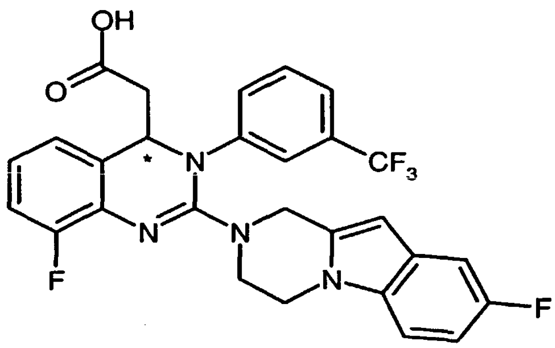

Ausgehend von 580 mg der Chinazolinylessigsäure aus Beispiel 7 werden nach Enantiomerentrennung (Methode 4) 235 mg des Enantiomers erhalten.
$^1$H-NMR (400 MHZ, $CD_3CN$): δ [ppm] = 7.65 (s, 1H); 7.43-7.35 (m, 3H); 7.24-7.16 (m, 2H); 7.05-6.86 (m, 4H); 6.20 (s, 1H); 5.24 (dd, 1H); 4.81 (dd, 1H), 4.09-4.03 (m, 3H); 3.85-3.76 (m, 2H); 2.71 (dd, 1H); 2.52 (dd, 1H).

**Allgemeine Arbeitsvorschrift [Q]: Reduktion der Dihydropyrazine zu Tetrahydropyrazinen**

0.14 mmol des Dihydropyrazines werden in 5 ml Eisessig gelöst und 0.56 mmol Natriumcyanoborhydrid zugesetzt. Nach einer Stunde bei Raumtemperatur (Reaktionskontrolle per DC) wird der Reaktionsansatz mit Wasser und Essigsäureethylester (je 10 ml) versetzt. Nach Phasentrennung wird die wässrige Phase mit Essigsäureethylester extrahiert (zweimal 10 ml) und die vereinigten organischen Extrakte mit gesättigter wässriger Ammoniumchlorid-Lösung (einmal 10 ml) und 0.5M Salzsäure (einmal 10 ml) gewaschen. Es wird über Natriumsulfat getrocknet und im Vakuum eingeengt. Das Produkt wird gegebenenfalls mittels präparativer HPLC (Methode 2) gereinigt.

**Beispiel 9**

[8-Fluor-3-[2-methoxy-5-(trifluormethyl)phenyl]-2-(9-methyl-3,4,10,10a-tetrahydropyrazino[1,2-a]indol-2(1H)-yl)-3,4-dihydro-chinazolin-4-yl]essigsäure

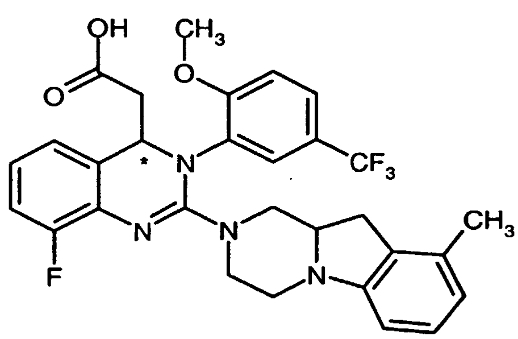

Ausgehend von 90 mg (0.16 mmol) des Enantiomers aus Beispiel 2 werden nach der allgemeinen Arbeitsvorschrift [Q] 94 mg (99 % d. Th.) Produkt erhalten.
HPLC (Methode 1): $R_t$ = 4.71 min

MS (ESIpos): m/z = 569 $(M+H)^+$
$^1$H-NMR (400 MHZ, $CD_3CN$): δ [ppm] = 7.61 (d, 1H); 7.19-6.90 (m, 6H); 6.47 (d, 1H); 6.24 (d, 1H); 5.08 (dd, 1H); 3.92-3.82 (m, 2H); 3.76 (s, 3H); 3.43-3.37 (m, 2H); 3.23-3.16 (m, 1H); 3.09-3.01 (m, 2H); 2.97-2.89 (m, 2H); 2.48-2.40 (m, 2H); 2.14 (s, 3H).

**Beispiel 10**

[8-Fluor-3-[2-methoxy-5-(trifluormethyl)phenyl]-2-(9-fluor-3,4,10,10a-tetrahydropyrazino[1,2-a]indol-2(1H)-yl)-3,4-dihydro-chinazolin-4-yl]essigsäure

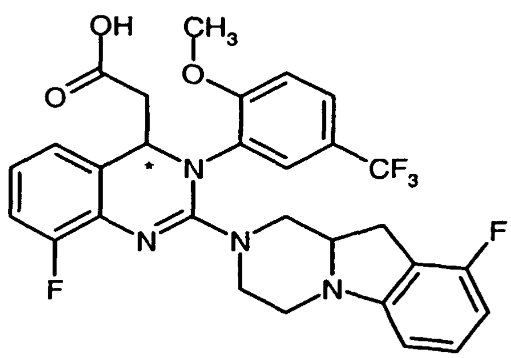

Ausgehend von 33 mg (0.06 mmol) des Enantiomers aus Beispiel 4 werden nach der allgemeinen Arbeitsvorschrift [Q] 14 mg (39 % d. Th.) Produkt erhalten.
HPLC (Methode 5): $R_t$ = 2.60 min
MS (ESIpos): m/z = 573 $(M+H)^+$
$^1$H-NMR (400 MHZ, $CD_3CN$): δ [ppm] = 7.60 (d, 1H); 7.16-6.96 (m, 6H); 6.37 (t, 1H); 6.24 (d, 1H); 5.05 (dd, 1H); 3.98-3.88 (m, 2H); 3.77 (s, 3H); 3.50-3.40 (m, 2H); 3.16-3.10 (m, 1H); 3.04-3.00 (m, 2H); 2.73-2.66 (m, 2H); 2.53 (dd, 2H).

**Beispiel 11**

[8-Fluor-3-[3-(trifluormethyl)phenyl]-2-(8-fluor-3,4,10,10a-tetrahydropyrazino[1,2-a]indol-2(1H)-yl)-3,4-dihydro-chinazolin-4-yl]essigsäure

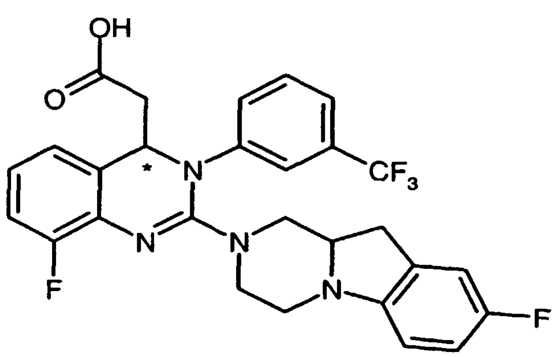

Ausgehend von 165 mg (0.28 mmol) des Enantiomers aus Beispiel 8 werden nach der allgemeinen Arbeitsvorschrift [Q] 155 mg (97 % d. Th.) Produkt erhalten.
HPLC (Methode 3): $R_t$ = 2.66 min
MS (ESIpos): m/z = 543 $(M+H)^+$

$^{1}$H-NMR (400 MHZ, $CD_3CN$): δ [ppm] = 7.77 (s, 1H); 7.60 (s, 3H); 7.23-7.20 (m, 2H); 7.08-7.06 (m, 1H); 6.83-6.77 (m, 2H); 6.36 (dd, 1H); 5.41 (dd, 1H); 3.51-3.42 (m, 2H); 3.30-2.25 (m, 1H); 3.06 (dd, 2H); 2.94 (dd, 2H); 2.74 (dd, 2H); 2.49-2.42 (m, 2H).

**Beispiel 12**

{8-Fluor-2-(8-fluor-3,4,10,10a-tetrahydropyrazino[1,2-a]indol-2(1H)-yl)-3-[3-(trifluormethyl)-phenyl]-3,4-dihydro-chinazolin-4-yl}essigsäure

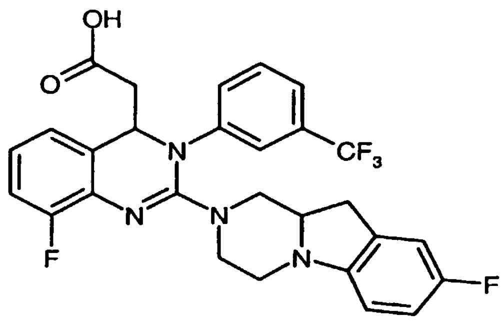

Ausgehend von 100 mg (0.19 mmol) des Indol Derivates aus Beispiel 7 werden nach der allgemeinen Arbeitsvorschrift [O] 40 mg (40 % d. Th.) Produkt erhalten.
HPLC (Methode 1): $R_t$= 4.62 min
MS (ESIpos): m/z = 543 $(M+H)^+$
$^{1}$H-NMR (400 MHZ, $CD_3CN$): δ [ppm] = 7.79 (s, 1H); 7.69-7.56 (m, 3H); 7.32-7.26 (m, 2H); 7. 13-7.08 (m, 1H); 6.88-6.76 (m, 2H); 6.40-6.38 (m, 1H); 5.42 (dd, 1H); 4.06-3.98 (m, 2H); 3.71-3.64 (m, 1H); 3.56-3.49 (m, 2H); 3.33-3.26 (m, 1H); 3.17-3.05 (m, 1H); 2.95-2.90 (m;2H); 2.84-2.78 (m, 1H); 2.53 (dd, 1H).

**Beispiel 13**

{8-Fluor-2-(9-methoxy-3,4,10,10a-tetrahydropyrazino[1,2-a]indol-2(1H)-yl)-3-[3-(trifluormethyl)-phenyl]-3,4-dihydro-chinazolin-4-yl}essigsäure

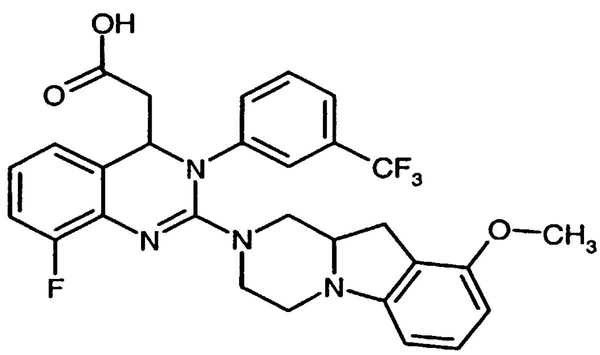

Ausgehend von 225 mg (0.41 mmol) des Indol Derivates aus Beispiel 5 werden nach der allgemeinen Arbeitsvorschrift [O] 50 mg (22 % d. Th.) Produkt erhalten.
HPLC (Methode 1): $R_t$ = 4.59 min
MS (ESIpos): m/z = 555 $(M+H)^+$

$^1$H-NMR (400 MHZ, $CD_3CN$): δ [ppm] = 7.59 (s, 1H); 7.47-7.36 (m, 4H); 7.04-6.88 (m, 4H); 6.31 (d, 1H); 6.12 (d, 1H); 5.24 (dd, 1H); 3.74 (s, 3H); 3.50-3.34 (m, 2H); 3.05-2.98 (m, 1H); 2.90-2.71 (m, 4H); 2.60-2.51 (m, 3H).

**Beispiel 14**

{8-Fluor-2-(8-fluor-3,4,10,10a-tetrahydropyrazino[1,2-a]indol-2(1H)-yl)-3-[2-methoxy-5-(trifluormethyl)phenyl]-3,4-dihydro-chinazolin-4-yl}essigsäure

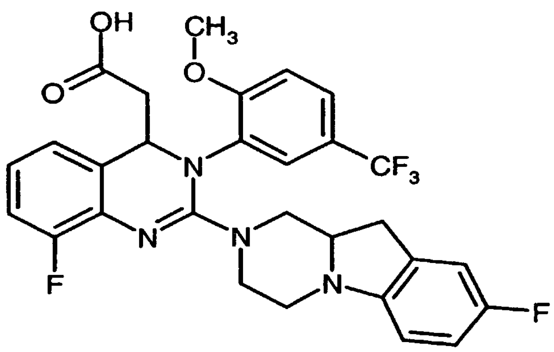

Ausgehend von 61 mg (0.10 mmol) des Indol Derivates aus Beispiel 6 werden nach der allgemeinen Arbeitsvorschrift [O] 20 mg (36 % d. Th.) Produkt erhalten.
HPLC (Methode 1): $R_t$= 4.56 min
MS (ESIpos): m/z = 573 $(M+H)^+$
$^1$H-NMR (400 MHZ, $CD_3CN$): δ [ppm] = 7.65 (d, 1H); 7.35-7.22 (m, 3H); 7.08-7.04 (m, 2H); 6.84 (d, 1H); 6.78-6.73 (m, 1H); 6.38-6.34 (m, 1H); 5.20 (dd, 1H); 4.05-3.80 (m, 4H); 4.57 (s, 3H); 3.40-2.77 (m, 6H); 2.97 (dd, 1H).

**Beispiel 15**

{8-Fluor-2-(8-fluor-3,4,10,10a-tetrahydropyrazino[1,2-a]indol-2(1H)-yl)-3-[2-methoxy-5-(trifluormethyl)phenyl]-3,4-dihydro-chinazolin-4-yl}essigsäure

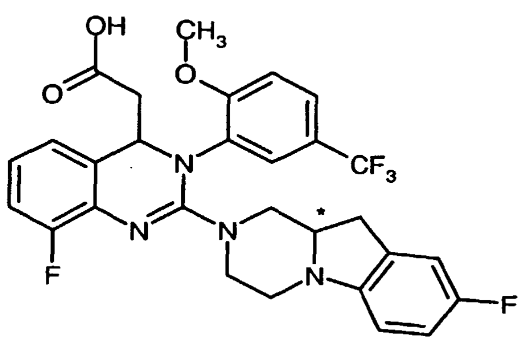

Ausgehend von 213 mg (0.36 mmol) des Methylesters aus Beispiel 50A werden nach der allgemeinen Arbeitsvorschrift [N] 207 mg (99 % d. Th.) Produkt erhalten.
HPLC (Methode 1): $R_t$ = 4.68 min
MS (ESIpos): m/z = 573 $(M+H)^+$
$^1$H-NMR (400 MHZ, $CD_3CN$): δ [ppm] = 7.48 (d, 1H); 7.33-7.29 (m, 1H); 7.14-7.05 (m, 1H); 7.02-6.98 (m, 1H); 6.90-6.82 (m, 4H); 6.36-6.30 (m, 1H); 4.89 (dd, 1H); 4.05-3.90 (m, 2H); 3.82 (s, 3H); 3.46-3.19 (m, 2H); 2.99-2.80 (m, 2H); 2.73-2.65 (m, 2H); 2.54-2.48 (m, 3H).

**Beispiel 16**

{8-Fluor-2-(8-fluor-3,4,10,10a-tetrahydropyrazino[1,2-a]indol-2(1H)-yl)-3-[2-methoxy-5-(trifluormethyl)phenyl]-3,4-dihydro-chinazolin-4-yl}essigsäure

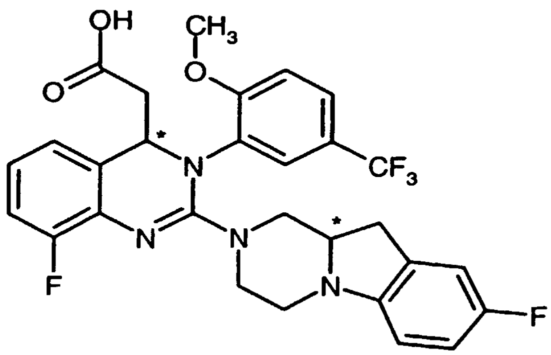

Ausgehend von 200 mg der Chinazolinylessigsäure aus Beispiel 15 werden nach Enantiomerentrennung (Methode 4) 70 mg des Enantiomers erhalten.
HPLC (Methode 3): $R_t$ = 2.46 min
MS (ESIpos): m/z = 573 $(M+H)^+$
$^1$H-NMR (400 MHZ, $CD_3CN$): δ [ppm] = 7.48 (d, 1H); 7.12-7.09 (m, 1H); 7.05-6.81 (m, 5H); 6.75-6.70 (m, 1H); 6.33-6.26 (m, 1H); 4.91 (dd, 1H); 4.05-3.89 (m, 2H); 3.82 (s, 3H); 3.29-3.19 (m, 2H); 3.03-2.97 (m, 1H); 2.91-2.82 (m, 2H); 2.77-2.70 (m, 1H); 2.56-2.44 (m, 3H).

Die Beispiele 17 bis 28 aus der nachfolgenden Tabelle können nach der allgemeinen Arbeitsvorschrift [P] oder [Q] hergestellt werden.

| Beispiel | Struktur | Molekulargewicht [g/mol] | Isomer | $R_t$ [min] | HPLC Methode | MS $(M+H)^+$ |
|---|---|---|---|---|---|---|
| 17 | 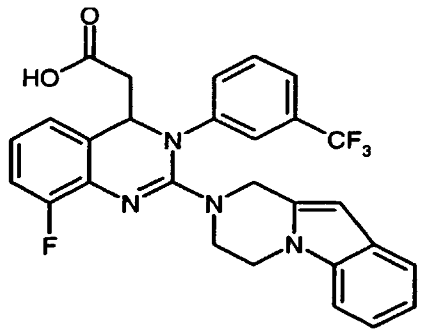 | 522.5 | Racemat | 4.63 | 1 | 523 |
| 18 | 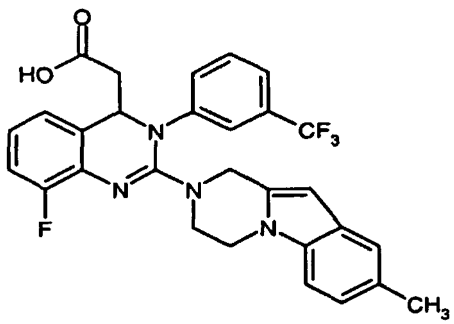 | 536.5 | Racemat | 4.74 | 1 | 537 |
| 19 | 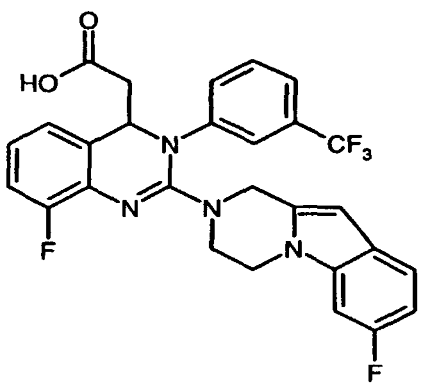 | 540.5 | Racemat | 4.59 | 1 | 541 |

(fortgesetzt)

| Beispiel | Struktur | Molekulargewicht [g/mol] | Isomer | $R_t$ [min] | HPLC Methode | MS $(M+H)^+$ |
|---|---|---|---|---|---|---|
| 20 | 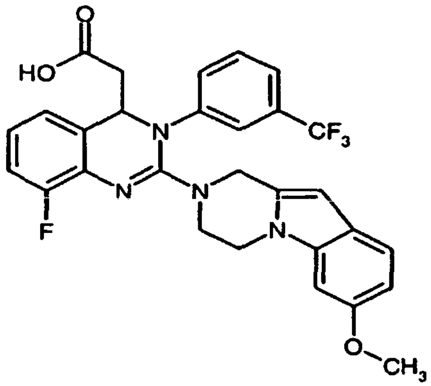 | 552.5 | Racemat | 4.59 | 1 | 553 |
| 21 | 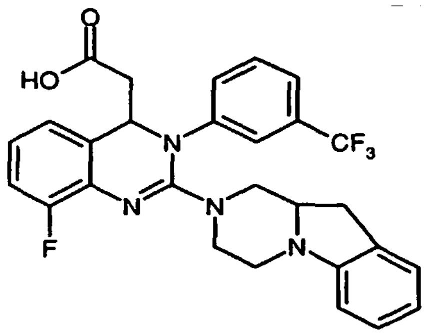 | 524.5 | Diasteremeren-Mischung | 4.56 | 1 | 525 |
| 22 | 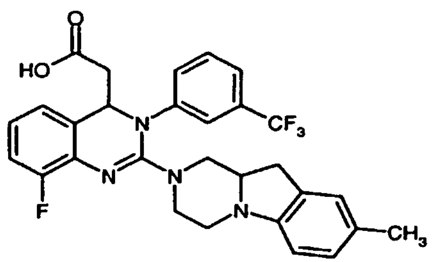 | 538.5 | Diastereomeren-Mischung | 4.63 | 1 | 539 |

(fortgesetzt)

| Beispiel | Struktur | Molekulargewicht [g/mol] | Isomer | $R_t$ [min] | HPLC Methode | MS $(M+H)^+$ |
|---|---|---|---|---|---|---|
| 23 | 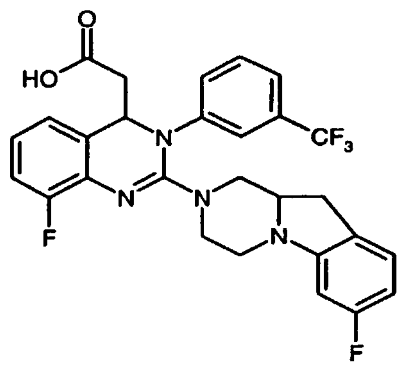 | 542.5 | Isomeren-Mischung | 4.61 | 1 | 543 |
| 24 | 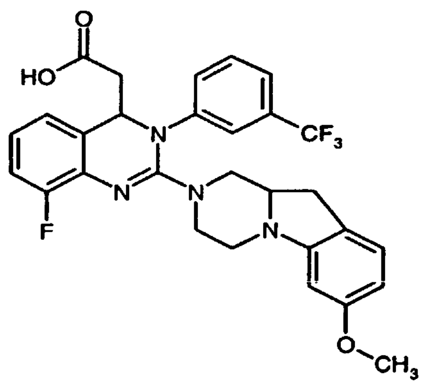 | 554.5 | Isomeren-Mischung | 4.62 | 1 | 555 |

(fortgesetzt)

| Beispiel | Struktur | Molekulargewicht [g/mol] | Isomer | $R_t$ [min] | HPLC Methode | MS $(M+H)^+$ |
|---|---|---|---|---|---|---|
| 25 | 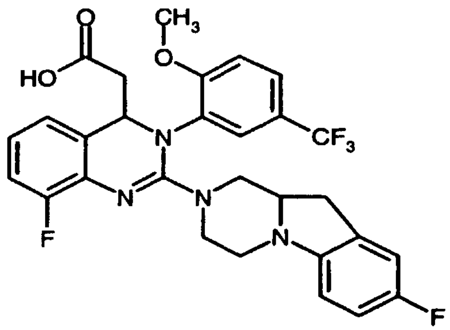 | 572.5 | Epimeren-Mischung B | 4.68 | 1 | 573 |
| 26 | 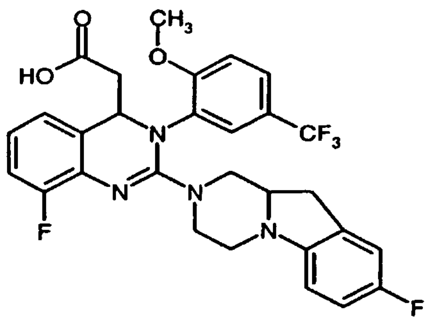 | 572.5 | Diastereomer 1 | 2.48 | 3 | 573 |
| 27 | 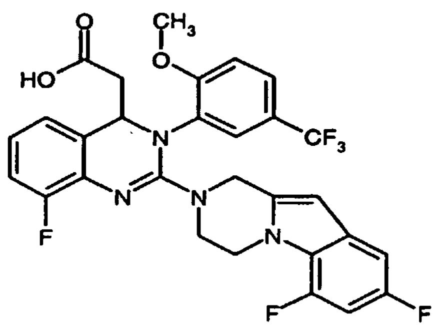 | 588.5 | Racemat | 4.66 | 1 | 589 |

(fortgesetzt)

| Beispiel | Struktur | Molekulargewicht [g/mol] | Isomer | $R_t$ [min] | HPLC Methode | MS $(M+H)^+$ |
|---|---|---|---|---|---|---|
| 28 | 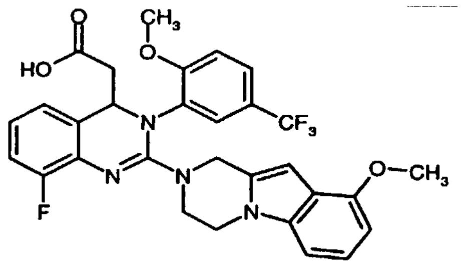 | 582.56 | Racemat | 4.56 | 1 | 583 |

**B. Bewertung der physiologischen Wirksamkeit**

Die in vitro-Wirkung der erfindungsgemäßen Verbindungen kann in folgenden Assays gezeigt werden:

**Anti-HCMV- (Anti-Humanes Cytomegalo-Virus) Zytopathogenitätstests**

Die Testverbindungen werden als 50 millimolare (mM) Lösungen in Dimethysulfoxid (DMSO) eingesetzt. Ganciclovir®, Foscarnet® und Cidofovir® dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x $10^4$ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5 % $CO_2$ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque Multiplier der Firma Technomara) visuell ausgewertet.

Die folgenden Daten können von den Testplatten ermittelt werden:

$CC_{50}$ (NHDF) = Substanzkonzentration in µM, bei der im Vergleich zur unbehandelten Zellkontrolle keine sichtbaren cytostatischen Effekte auf die Zellen erkennbar sind;

$EC_{50}$ (HCMV) = Substanzkonzentration in µM, die den CPE (cytopathischen Effekt) um 50 % im Vergleich zur unbehandelten Viruskontrolle hemmt;

**SI (Selektivitätsindex) = $CC_{50}$ (NHDF) / $EC_{50}$ (HCMV).**

Repräsentative in-vitro-Wirkdaten für die erfindungsgemäßen Verbindungen sind in Tabelle A wiedergegeben:

**Tabelle A**

| Beispiel-Nr. | NHDF $CC_{50}$ [µM] | HCMV $EC_{50}$ [µM] | SI HCMV |
|---|---|---|---|
| 2 | 21 | 0.09 | 233 |
| 4 | 14 | 0.12 | 117 |
| 9 | 24 | 0.04 | 600 |
| 16 | 21 | 0.02 | 1050 |

Die Eignung der erfindungsgemäßen Verbindungen zur Behandlung von HCMV-Infektionen kann im folgenden Tiermodell gezeigt werden:

**HCMV Xenograft-Geloam®-Modell**

Tiere:

3-4 Wochen alte weibliche immundefiziente Mäuse (16-18 g), Fox Chase SCID oder Fox Chase SCID-NOD oder SCID-beige werden von kommerziellen Züchtern (Taconic M+B, Jackson, USA) bezogen. Die Tiere werden unter sterilen Bedingungen (einschließlich Streu und Futter) in Isolatoren gehalten.

Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01-0.03 werden die virusinfizierten Zellen 5-10 Tage später geerntet und in Gegenwart von Minimal Essential

Medium (MEM), 10 % foetalem Kälberserum (FKS) mit 10 % DMSO bei -40˚C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot.

Vorbereitung der Schwämme, Transplantation, Behandlung und Auswertung:

1x1x1 cm große Kollagenschwämme (Gelfoam®; Fa. Peasel & Lorey, Best.-Nr. 407534; K.T. Chong et al., Abstracts of 39$^{th}$ Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM + 10 % FKS aufbewahrt. 1 x $10^6$ virusinfizierte NHDF-Zellen (Infektion mit HCMV-Davis oder HCMV AD169 M.O.I = 0.03) werden 3 Stunden nach Infektion abgelöst und in 20 µl MEM, 10 % FKS auf einen feuchten Schwamm getropft. Ca. 16 Stunden später werden die mit den infizierten Zellen beladenen Schwämme mit 25 µl PBS / 0.1 % BSA / 1 mM DTT mit 5 ng/µl basic Fibroblast Growth Factor (bFGF) inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber verschlossen. 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (14 Uhr) peroral mit Substanz behandelt. Die Tagesdosis beträgt beispielsweise 3 oder 10 oder 30 oder 60 oder 100 mg/kg Körpergewicht, das Applikationsvolumen 10 ml/kg Körpergewicht. Die Formulierung der Substanzen erfolgt in Form einer 0.5 %-igen Tylosesuspension mit 2 % DMSO oder einer 0.5 %-igen Tylosesuspension. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U/1.5 ml) aus dem Schwamm freigesetzt und in Gegenwart von MEM, 10 % foetalem Kälberserum, 10 % DMSO bei -140˚C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wird die Anzahl infizierter Zellen bzw. infektiöser Viruspartikel (infectious center assay) nach Substanzbehandlung im Vergleich zur placebobehandelten Kontrollgruppe.

**CYP-Inhibitions-Assay**

Zur Untersuchung der mechanism-based (irreversiblen) Inhibition von CYP3A4 wird die Testsubstanz in verschiedenen Konzentrationen mit Humanlebermikrosomen (2mg/ml mikrosomales Protein) in Kaliumphosphatpuffer pH 7.4 unter Zusatz von NADPH-generierendem System (NADP+, Glucose-6-phosphat und Glucose-6-phosphatdehydrogenase) bei 37˚C inkubiert. Zu verschiedenen Zeitpunkten werden 2 Aliqouts aus der Inkubation entnommen.

Das erste Aliqout wird 1:50 in eine neue Inkubationslösung (Phosphatpuffer, NADPH-generierendes System und 10 µM Midazolam) für weitere 10 min bei 37˚C inkubiert. Danach wird die Inkubation mit Acetonitril auf Eis gestoppt, in der Zentrifuge bei 15000g das Protein pelletiert, und der Überstand mit HPLC/MS nach Standardmethoden auf die Bildung von 1'-Hydroxymidazolam analysiert.

Das zweite Aliquot wird mit Acetonitril auf Eis gestoppt und mit HPLC/UV/MS auf verbleibenden Testsubstanz analysiert.

Aus beiden analytischen Datensätzen werden für irreversible Inhibition typische Parameter ($k_{inact}$, $K_i$ und partition ratio r) bestimmt und die Testsubstanz damit bewertet (vgl. A. Madan, et al., in A.D. Rodrigues (ed.) "Drug-Drug Interaction" in "Drugs and the Pharmaceutical Science", Vol. 116, , ISBN 0-8247-0283.2, Marcel Dekker Inc., New York, 2002.).

**C. Ausführungsbeispiele für pharmazeutische Zusammensetzungen**

Die erfindungsgemäßen Verbindungen können folgendermaßen in pharmazeutische Zubereitungen überführt werden:

**Tablette:**

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Suspension:**

Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

**Intravenös applizierbare Lösung:**

Zusammensetzung:

10-200 mg der Verbindung von Beispiel 1, 15 g Polyethylenglykol 400 und 250 g Wasser für Injektionszwecke.

Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 μm) und unter aseptischen Bedingungen in hitzesterilisierte Infusionsflaschen abgefüllt. Diese werden mit Infusionsstopfen und Bördelkappen verschlossen.

**Patentansprüche**

1. Verbindung der Formel

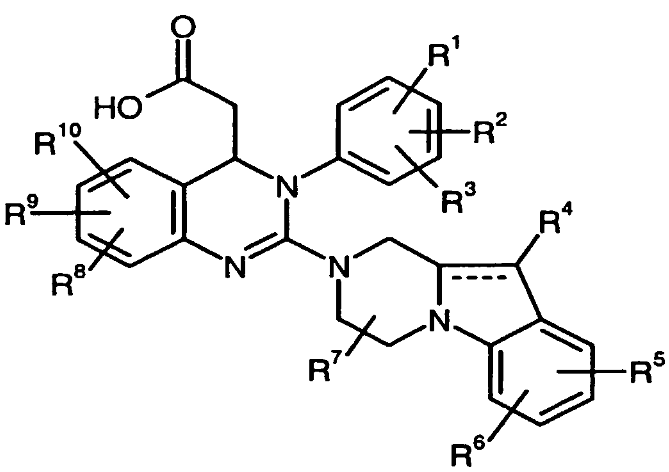

(I),

in welcher

........ für eine Einfach- oder Doppelbindung steht,
$R^1$ für Wasserstoff, Amino, Alkyl, Alkoxy, Alkylamino, Alkylthio, Cyano, Halogen, Nitro oder Trifluormethyl steht,

$R^2$ für Wasserstoff, Alkyl, Alkoxy, Alkylthio, Cyano, Halogen, Nitro oder Trifluormethyl steht,
$R^3$ für Amino, Alkyl, Alkoxy, Alkylamino, Alkylthio, Cyano, Halogen, Nitro, Trifluormethyl, Alkylsulfonyl oder Alkylaminosulfonyl steht

oder
einer der Reste $R^1$, $R^2$ und $R^3$ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,

$R^4$ für Wasserstoff oder Alkyl steht,
$R^5$ für Wasserstoff, Alkyl, Alkoxy, Formyl, Carboxy, Alkylcarbonyl, Alkoxycarbonyl, Trifluormethyl, Halogen, Cyano, Hydroxy, Amino, Alkylamino, Aminocarbonyl oder Nitro steht,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,
$R^6$ für Wasserstoff, Alkyl, Alkoxy, Formyl, Carboxy, Alkylcarbonyl, Alkoxycarbonyl, Trifluormethyl, Halogen, Cyano, Hydroxy, Amino, Alkylamino, Aminocarbonyl oder Nitro steht,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl

oder

$R^5$ und $R^6$ zusammen mit den Kohlenstoffatomen, an die sie gebunden sind, ein 1,3-Dioxolan, einen Cyclopentan-Ring oder einen Cyclohexan-Ring bilden,
$R^7$ für Wasserstoff oder Alkyl steht,
$R^8$ für Wasserstoff, Alkyl, Alkoxy, Alkylamino, Alkylthio, Formyl, Carboxy, Aminocarbonyl, Alkylaminocarbonyl, Alkylcarbonyl, Alkoxycarbonyl, Trifluormethyl, Halogen, Cyano, Hydroxy, Nitro oder einen über Stickstoff gebundenen 5- bis 7-gliedrigen Heterocyclus steht,
$R^9$ für Wasserstoff, Alkyl, Alkoxy, Alkylthio, Formyl, Carboxy, Alkylcarbonyl, Alkoxycarbonyl, Trifluormethyl, Halogen, Cyano, Hydroxy oder Nitro steht
$R^{10}$ für Wasserstoff, Alkyl, Alkoxy, Alkylthio, Formyl, Carboxy, Alkylcarbonyl, Alkoxycarbonyl, Trifluormethyl, Halogen, Cyano, Hydroxy oder Nitro steht,

oder eines ihrer Salze, ihrer Solvate oder der Solvate ihrer Salze.

**2.** Verbindung nach Anspruch 1, **dadurch gekennzeichnet, dass**

........ für eine Einfach- oder Doppelbindung steht,
$R^1$ für Wasserstoff, $C_1$-$C_6$-Alkyl, $C_1$-$C_6$-Alkoxy, $C_1$-$C_6$-Alkylthio, Fluor oder Chlor steht,
$R^2$ für Wasserstoff, $C_1$-$C_6$-Alkyl, $C_1$-$C_6$-Alkoxy, $C_1$-$C_6$-Alkylthio, Fluor oder Chlor steht,
$R^3$ für $C_1$-$C_4$-Alkyl, Cyano, Fluor, Chlor, Nitro oder Trifluormethyl steht,

oder
einer der Reste $R^1$, $R^2$ und $R^3$ für Wasserstoff, $C_1$-$C_3$-Alkyl, $C_1$-$C_3$-Alkoxy, Cyano, Halogen, Nitro oder Trifluormethyl steht und die anderen beiden zusammen mit den Kohlenstoffatomen, an die sie gebunden sind, einen Cyclopentan-Ring oder einen Cyclohexan-Ring bilden,

$R^4$ für Wasserstoff steht,
$R^5$ für Wasserstoff, $C_1$-$C_6$-Alkyl, $C_1$-$C_6$-Alkoxy, Carboxy, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino oder Nitro steht,
$R^6$ für Wasserstoff, $C_1$-$C_6$-Alkyl, $C_1$-$C_6$-Alkoxy, Carboxy, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino oder Nitro steht

oder

$R^5$ und $R^6$ zusammen mit den Kohlenstoffatomen, an die sie gebunden sind, ein 1,3-Dioxolan bilden,
$R^7$ für Wasserstoff oder Methyl steht,
$R^8$ für $C_1$-$C_3$-Alkyl, $C_1$-$C_3$-Alkoxy, Carboxy, Aminocarbonyl, $C_1$-$C_3$-Alkylaminocarbonyl, Trifluormethyl, Fluor, Chlor, Cyano, Hydroxy oder Nitro steht,

$R^9$ für Wasserstoff, $C_1$-$C_3$-Alkyl, $C_1$-$C_3$-Alkoxy, Fluor, Chlor, Cyano oder Hydroxy steht
und
$R^{10}$ für Wasserstoff, $C_1$-$C_3$-Alkyl, $C_1$-$C_3$-Alkoxy, Fluor, Chlor, Cyano oder Hydroxy steht.

**3.** Verbindung nach einem der Ansprüche 1 oder 2, **dadurch gekennzeichnet, dass**

........ für eine Einfach- oder Doppelbindung steht,
$R^1$ für Wasserstoff, Methyl, Methoxy, Methylthio, Fluor oder Chlor steht,
$R^2$ für Wasserstoff steht,
$R^3$ für Methyl, Cyano, Fluor, Chlor, Nitro oder Trifluormethyl steht,
$R^4$ für Wasserstoff steht,
$R^5$ für Wasserstoff, Methyl, Methoxy, Fluor oder Chlor steht,
$R^6$ für Wasserstoff, Methyl, Methoxy, Fluor oder Chlor steht,
$R^7$ für Wasserstoff steht,
$R^8$ für Aminocarbonyl, Fluor, Chlor, Cyano oder Hydroxy steht,
$R^9$ für Wasserstoff steht
und
$R^{10}$ für Wasserstoff steht.

**4.** Verbindung nach einem der Ansprüche 1 bis 3, **dadurch gekennzeichnet, dass** ........ für eine Einfachbindung steht.

**5.** Verbindung nach einem der Ansprüche 1 bis 4, **dadurch gekennzeichnet, dass** $R^1$ für Wasserstoff, Methyl, Methoxy oder Fluor steht.

**6.** Verbindung nach einem der Ansprüche 1 bis 5, **dadurch gekennzeichnet, dass** $R^1$ für Methoxy steht.

**7.** Verbindung nach einem der Ansprüche 1 bis 6, **dadurch gekennzeichnet, dass** $R^1$ über die ortho-Position zur Verknüpfungsstelle des Phenylrings an den Phenylring gebunden ist.

**8.** Verbindung nach einem der Ansprüche 1, 2 oder 4 bis 7, **dadurch gekennzeichnet, dass** $R^2$ für Wasserstoff steht.

**9.** Verbindung nach einem der Ansprüche 1, 2 oder 4 bis 8, **dadurch gekennzeichnet, dass** $R^3$ für Trifluormethyl, Chlor, Methyl, iso-Propyl oder *tert.*-Butyl steht.

**10.** Verbindung nach einem der Ansprüche 1 bis 9, **dadurch gekennzeichnet, dass** $R^3$ für Trifluormethyl, Chlor oder Methyl steht.

**11.** Verbindung nach einem der Ansprüche 1 bis 10, **dadurch gekennzeichnet, dass** $R^1$ über die ortho-Position zur Verknüpfungsstelle des Phenylrings an den Phenylring gebunden ist und $R^3$ über die $R^1$ gegenüberliegende meta-Position zur Verknüpfungsstelle des Phenylrings an den Phenylring gebunden ist.

**12.** Verbindung nach einem der Ansprüche 1 oder 4 bis 11, **dadurch gekennzeichnet, dass** $R^4$ für Wasserstoff steht.

**13.** Verbindung nach einem der Ansprüche 1, 2 oder 4 bis 12, **dadurch gekennzeichnet, dass** $R^5$ für Wasserstoff, Methyl, Methoxy, Fluor oder Chlor steht.

**14.** Verbindung nach einem der Ansprüche 1 bis 13, **dadurch gekennzeichnet, dass** $R^6$ für Wasserstoff, Methyl, Methoxy oder Fluor steht.

**15.** Verbindung nach einem der Ansprüche 1, 2 oder 4 bis 14, **dadurch gekennzeichnet, dass** $R^7$ für Wasserstoff steht.

**16.** Verbindung nach einem der Ansprüche 1 bis 15, **dadurch gekennzeichnet, dass** $R^8$ für Fluor steht.

**17.** Verbindung nach einem der Ansprüche 1, 2 oder 4 bis 16, **dadurch gekennzeichnet, dass** $R^9$ für Wasserstoff steht.

**18.** Verbindung nach einem der Ansprüche 1, 2 oder 4 bis 17, **dadurch gekennzeichnet, dass** $R^{10}$ für Wasserstoff, Methyl oder Fluor steht.

**19.** Verfahren zur Herstellung einer Verbindung der Formel (I) nach Anspruch 1, **dadurch gekennzeichnet, dass** nach Verfahren [A] eine Verbindung der Formel

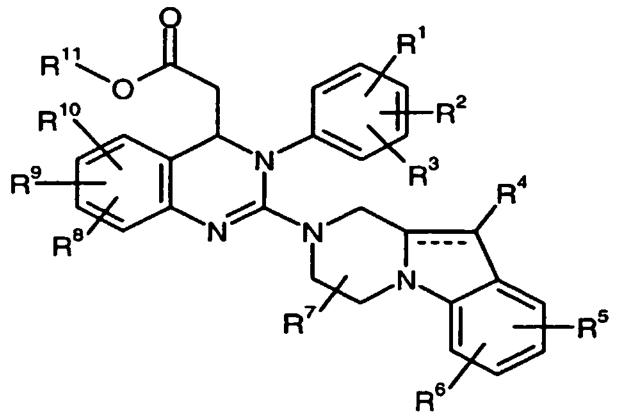

(II),

in welcher
......, $R^1$, $R^2$, $R^3$, $R^4$, $R^5$, $R^6$, $R^7$, $R^8$, $R^9$ und $R^{10}$ die in Anspruch 1 angegebene Bedeutung haben
und
$R^{11}$ für Alkyl, bevorzugt Methyl oder Ethyl, steht,
mit Basen umgesetzt werden,
oder
nach Verfahren [B] eine Verbindung der Formel

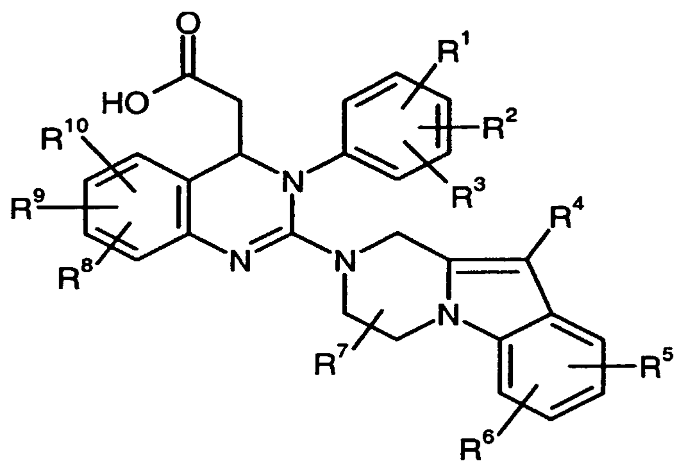

(Ib),

in welcher
$R^1$, $R^2$, $R^3$, $R^4$, $R^5$, $R^6$, $R^7$, $R^8$, $R^9$ und $R^{10}$ die in Anspruch 1 angegebene Bedeutung haben,
durch Umsetzung mit Reduktionsmitteln zu Verbindungen der Formel

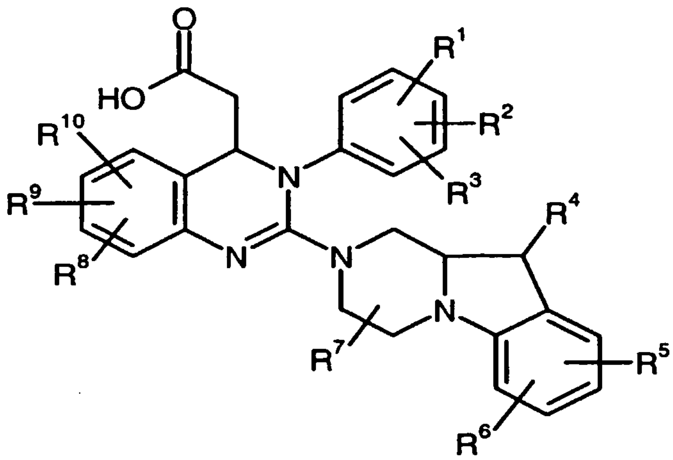

(Ic),

in welcher
$R^1$, $R^2$, $R^3$, $R^4$, $R^5$, $R^6$, $R^7$, $R^8$, $R^9$ und $R^{10}$ die in Anspruch 1 angegebene Bedeutung haben, umgesetzt werden.

**20.** Verbindung nach einem der Ansprüche 1 bis 18 zur Behandlung und/oder Prophylaxe von Krankheiten.

**21.** Arzneimittel enthaltend eine Verbindung nach einem der Ansprüche 1 bis 18 in Kombination mit einem inerten, nichttoxischen, pharmazeutisch geeigneten Hilfsstoff.

**22.** Verwendung einer Verbindung nach einem der Ansprüche 1 bis 18 zur Herstellung eines Arzneimittels zur Behandlung und/oder Prophylaxe von Virusinfektionen.

**23.** Verwendung nach Anspruch 22, **dadurch gekennzeichnet, dass** die Virusinfektion eine Infektion mit dem humanen Cytomegalovirus (HCMV) oder einem anderen Vertreter der Gruppe der Herpes viridae ist.

**24.** Arzneimittel nach Anspruch 21 zur Behandlung und/oder Prophylaxe von Virusinfektionen.

**Claims**

**1.** Compound of the formula

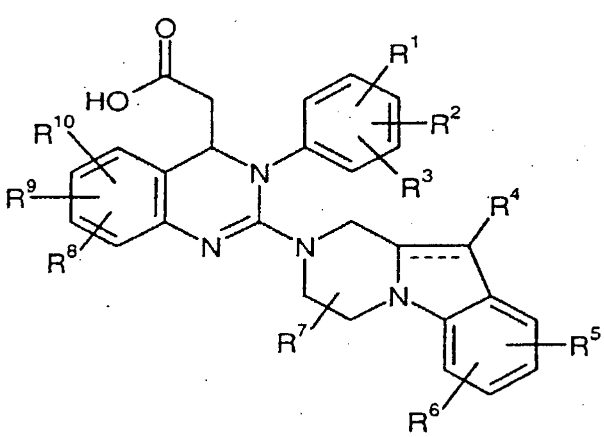

(I),

in which

......... represents a single or double bond,
$R^1$ represents hydrogen, amino, alkyl, alkoxy, alkylamino, alkylthio, cyano, halogen, nitro or trifluoromethyl,
$R^2$ represents hydrogen, alkyl, alkoxy, alkylthio, cyano, halogen, nitro or trifluoromethyl,
$R^3$ represents amino, alkyl, alkoxy, alkylamino, alkylthio, cyano, halogen, nitro, trifluoromethyl, alkylsulphonyl or alkylaminosulphonyl

or
one of the radicals $R^1$, $R^2$ and $R^3$ represents hydrogen, alkyl, alkoxy, cyano, halogen, nitro or trifluoromethyl and the other two together with the carbon atoms to which they are attached form a 1,3-dioxolane, a cyclopentane ring or a cyclohexane ring,

$R^4$ represents hydrogen or alkyl,
$R^5$ represents hydrogen, alkyl, alkoxy, formyl, carboxyl, alkylcarbonyl, alkoxycarbonyl, trifluoromethyl, halogen, cyano, hydroxyl, amino, alkylamino, aminocarbonyl or nitro,
where alkyl may be substituted by 1 to 3 substituents, where the substituents are selected independently of one another from the group consisting of halogen, amino, alkylamino, hydroxyl and aryl,
$R^6$ represents hydrogen, alkyl, alkoxy, formyl, carboxyl, alkylcarbonyl, alkoxycarbonyl, trifluoromethyl, halogen, cyano, hydroxyl, amino, alkylamino, aminocarbonyl or nitro,
where alkyl may be substituted by 1 to 3 substituents, where the substituents are selected independently of one another from the group consisting of halogen, amino, alkylamino, hydroxyl and aryl

or

$R^5$ and $R^6$ together with the carbon atoms to which they are attached form a 1,3-dioxolane, a cyclopentane ring or a cyclohexane ring,
$R^7$ represents hydrogen or alkyl,
$R^8$ represents hydrogen, alkyl, alkoxy, alkylamino, alkylthio, formyl, carboxyl, aminocarbonyl, alkylaminocarbonyl, alkylcarbonyl, alkoxycarbonyl, trifluoromethyl, halogen, cyano, hydroxyl, nitro or a 5- to 7-membered heterocycle which is attached via nitrogen,
$R^9$ represents hydrogen, alkyl, alkoxy, alkylthio, formyl, carboxyl, alkylcarbonyl, alkoxycarbonyl, trifluoromethyl, halogen, cyano, hydroxyl or nitro,
$R^{10}$ represents hydrogen, alkyl, alkoxy, alkylthio, formyl, carboxyl, alkylcarbonyl, alkoxycarbonyl, trifluoromethyl, halogen, cyano, hydroxyl or nitro,

or a salt, a solvate or a solvate of a salt thereof.

**2.** Compound according to Claim 1, **characterized in that**

........ represents a single or double bond,
$R^1$ represents hydrogen, $C_1$-$C_6$-alkyl, $C_1$-$C_6$-alkoxy, $C_1$-$C_6$-alkylthio, fluorine or chlorine,
$R^2$ represents hydrogen, $C_1$-$C_6$-alkyl, $C_1$-$C_6$-alkoxy, $C_1$-$C_6$-alkylthio, fluorine or chlorine,
$R^3$ represents $C_1$-$C_4$-alkyl, cyano, fluorine, chlorine, nitro or trifluoromethyl,

or
one of the radicals $R^1$, $R^2$ and $R^3$ represents hydrogen, $C_1$-$C_3$-alkyl, $C_1$-$C_3$-alkoxy, cyano, halogen, nitro or trifluoromethyl and the other two together with the carbon atoms to which they are attached form a cyclopentane ring or a cyclohexane ring,

$R^4$ represents hydrogen,
$R^5$ represents hydrogen, $C_1$-$C_6$-alkyl, $C_1$-$C_6$-alkoxy, carboxyl, $C_1$-$C_6$-alkylcarbonyl, $C_1$-$C_6$-alkoxycarbonyl, trifluoromethyl, fluorine, chlorine, bromine, cyano, hydroxyl, amino, $C_1$-$C_6$-alkylamino or nitro,
$R^6$ represents hydrogen, $C_1$-$C_6$-alkyl, $C_1$-$C_6$-alkoxy, carboxyl, $C_1$-$C_6$-alkylcarbonyl, $C_1$-$C_6$-alkoxycarbonyl, trifluoromethyl, fluorine, chlorine, bromine, cyano, hydroxyl, amino, $C_1$-$C_6$-alkylamino or nitro
or
$R^5$ and $R^6$ together with the carbon atoms to which they are attached form a 1,3-dioxolane,
$R^7$ represents hydrogen or methyl,

$R^8$ represents $C_1$-$C_3$-alkyl, $C_1$-$C_3$-alkoxy, carboxyl, aminocarbonyl, $C_1$-$C_3$-alkylaminocarbonyl, trifluoromethyl, fluorine, chlorine, cyano, hydroxyl or nitro,
$R^9$ represents hydrogen, $C_1$-$C_3$-alkyl, $C_1$-$C_3$-alkoxy, fluorine, chlorine, cyano or hydroxyl
and
$R^{10}$ represents hydrogen, $C_1$-$C_3$-alkyl, $C_1$-$C_3$-alkoxy, fluorine, chlorine, cyano or hydroxyl.

**3.** Compound according to any of Claims 1 or 2, **characterized in that**

<u>........</u> represents a single or double bond,
$R^1$ represents hydrogen, methyl, methoxy, methylthio, fluorine or chlorine,
$R^2$ represents hydrogen,
$R^3$ represents methyl, cyano, fluorine, chlorine, nitro or trifluoromethyl,
$R^4$ represents hydrogen,
$R^5$ represents hydrogen, methyl, methoxy, fluorine or chlorine,
$R^6$ represents hydrogen, methyl, methoxy, fluorine or chlorine,
$R^7$ represents hydrogen,
$R^8$ represents aminocarbonyl, fluorine, chlorine, cyano or hydroxyl,
$R^9$ represents hydrogen
and
$R^{10}$ represents hydrogen.

**4.** Compound according to any of Claims 1 to 3, **characterized in that** <u>........</u> represents a single bond.

**5.** Compound according to any of Claims 1 to 4, **characterized in that** $R^1$ represents hydrogen, methyl, methoxy or fluorine.

**6.** Compound according to any of Claims 1 to 5, **characterized in that** $R^1$ represents methoxy.

**7.** Compound according to any of Claims 1 to 6, **characterized in that** $R^1$ is attached to the phenyl ring via the position ortho to the point of attachment of the phenyl ring.

**8.** Compound according to any of Claims 1, 2 or 4 to 7, **characterized in that** $R^2$ represents hydrogen.

**9.** Compound according to any of Claims 1, 2, or 4 to 8, **characterized in that** $R^3$ represents trifluoromethyl, chlorine, methyl, isopropyl or tert-butyl.

**10.** Compound according to any of Claims 1 to 9, **characterized in that** $R^3$ represents trifluoromethyl, chlorine or methyl.

**11.** Compound according to any of Claims 1 to 10, **characterized in that** $R^1$ is attached to the phenyl ring via the position ortho to the point of attachment of the phenyl ring and $R^3$ is attached to the phenyl ring via the position meta to the point of attachment of the phenyl ring, which position is opposite to that of $R^1$.

**12.** Compound according to any of Claims 1 or 4 to 11, **characterized in that** $R^4$ represents hydrogen.

**13.** Compound according to any of Claims 1, 2 or 4 to 12, **characterized in that** $R^5$ represents hydrogen, methyl, methoxy, fluorine or chlorine.

**14.** Compound according to any of Claims 1 to 13, **characterized in that** $R^6$ represents hydrogen, methyl, methoxy or fluorine.

**15.** Compound according to any of Claims 1, 2 or 4 to 14, **characterized in that** $R^7$ represents hydrogen.

**16.** Compound according to any of Claims 1 to 15, **characterized in that** $R^8$ represents fluorine.

**17.** Compound according to any of Claims 1, 2 or 4 to 16, **characterized in that** $R^9$ represents hydrogen.

**18.** Compound according to any of Claims 1, 2 or 4 to 17, **characterized in that** $R^{10}$ represents hydrogen, methyl or fluorine.

**19.** Process for preparing a compound of the formula (I) according to Claim 1, **characterized in that**, according to process [A], a compound of the formula

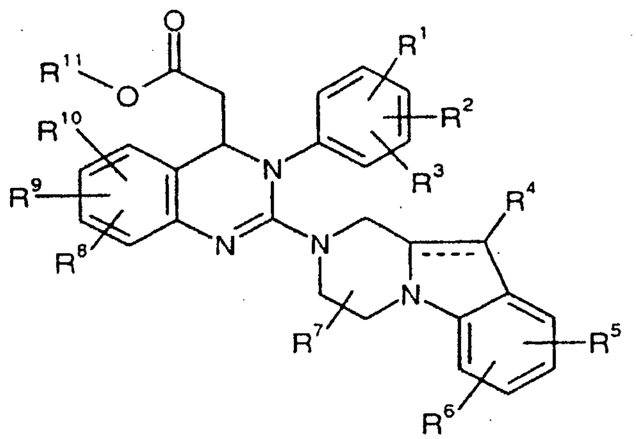

(II),

in which
........, $R^1$, $R^2$, $R^3$, $R^4$, $R^5$, $R^6$, $R^7$, $R^8$, $R^9$ and $R^{10}$ are as defined in Claim 1,
and
$R^{11}$ represents alkyl, preferably methyl or ethyl,
is reacted with bases,
or,
according to process [B], a compound of the formula

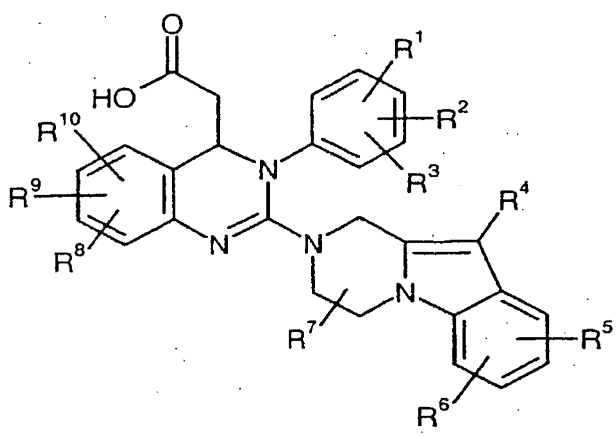

(Ib),

in which
$R^1$ $R^2$, $R^3$, $R^4$, $R^5$, $R^6$ $R^7$, $R^8$, $R^9$ and $R^{10}$ are as defined in Claim 1 is,
by reaction with reducing agents, converted into a compound of the formula

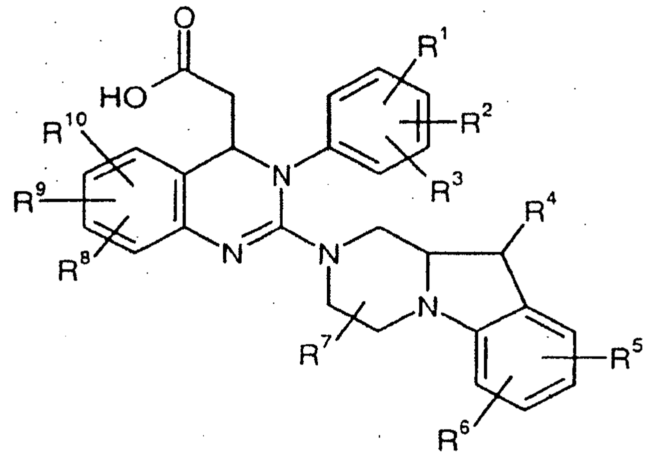

(Ic),

in which
$R^1$, $R^2$, $R^3$, $R^4$, $R^5$, $R^6$, $R^7$, $R^8$, $R^9$ and $R^{10}$ are as defined in Claim 1.

**20.** Compound according to any of Claims 1 to 18 for the treatment and/or the prophylaxis of diseases.

**21.** Medicament, comprising a compound according to any of Claims 1 to 18 in combination with an inert nontoxic pharmaceutically acceptable auxiliary.

**22.** Use of a compound according to any of Claims 1 to 18 for preparing a medicament for the treatment and/or prophylaxis of viral infections.

**23.** Use according to Claim 22, **characterized in that** the viral infection is an infection with the human cytomegalovirus (HCMV) or another representative of the group of the Herpes viridae.

**24.** Medicament according to Claim 21 for the treatment and/or prophylaxis of viral infections.

**Revendications**

**1.** Composé de formule I

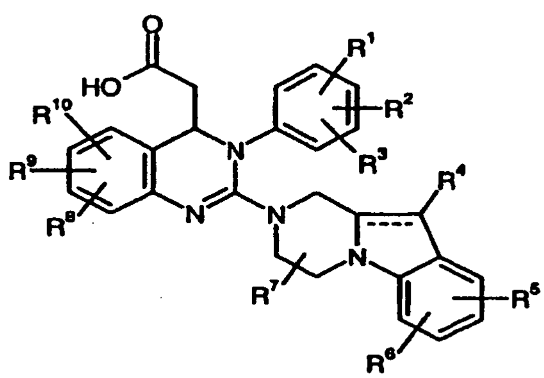

(I),

dans laquelle

..... désigne une liaison simple ou une liaison double,
$R^1$ représente l'hydrogène, un groupe amino, un reste alkyle, alkoxy, alkylamino, alkylthio, un groupe cyano,

un halogène, un groupe nitro ou un reste trifluorométhyle,
$R^2$ représente l'hydrogène, un reste alkyle, alkoxy, alkylthio, un groupe cyano, un halogène, un groupe nitro ou un reste trifluorométhyle,
$R^3$ représente un groupe amino, un reste alkyle, alkoxy, alkylamino, alkylthio, un groupe cyano, un halogène, un groupe nitro, un reste trifluorométhyle, alkylsulfonyle ou alkylaminosulfonyle,

ou bien
l'un des restes $R^1$, $R^2$ et $R^3$ représente l'hydrogène, un reste alkyle, alkoxy, un groupe cyano, un halogène, un groupe nitro ou un reste trifluorométhyle et les deux autres restes forment, conjointement avec les atomes d'azote auxquels ils sont liés, un groupe 1,3-dioxolanne, un noyau cyclopentane ou un noyau cyclohexane,

$R^4$ représente l'hydrogène ou un reste alkyle,
$R^5$ représente l'hydrogène, un reste alkyle, alkoxy, formyle, carboxy, alkylcarbonyle, alkoxycarbonyle, trifluorométhyle, un halogène, un groupe cyano, hydroxy, amino, un reste alkylamino, aminocarbonyle ou un groupe nitro,
un reste alkyle pouvant être substitué avec 1 à 3 substituants qui sont choisis indépendamment les uns des autres dans le groupe constitué de radicaux halogéno, amino, alkylamino, hydroxy et aryle,
$R^6$ représente l'hydrogène, un reste alkyle, alkoxy, formyle, carboxy, alkylcarbonyle, alkoxycarbonyle, trifluorométhyle, un halogène, un groupe cyano, hydroxy, amino, un reste alkylamino, aminocarbonyle ou un groupe nitro,
un reste alkyle pouvant être substitué avec 1 à 3 substituants choisis indépendamment les uns des autres dans le groupe constitué des radicaux halogéno, amino, alkylamino, hydroxy et aryle,

ou bien

$R^5$ et $R^6$ forment, conjointement avec les atomes de carbone auxquels ils sont liés, un groupe 1,3-dioxolanne, un noyau cyclopentane ou un noyau cyclohexane,
$R^7$ représente l'hydrogène ou un reste alkyle,
$R^8$ représente l'hydrogène, un reste alkyle, alkoxy, alkylamino, alkylthio, formyle, carboxy, aminocarbonyle, alkylaminocarbonyle, alkylcarbonyle, alkoxycarbonyle, trifluorométhyle, un halogène, un groupe cyano, hydroxy, nitro ou un hétérocycle pentagonal à heptagonal lié par l'intermédiaire d'azote,
$R^9$ représente l'hydrogène, un reste alkyle, alkoxy, alkylthio, formyle, carboxy, alkylcarbonyle, alkoxycarbonyle, trifluorométhyle, un halogène, un groupe cyano, hydroxy ou nitro,
$R^{10}$ représente l'hydrogène, un reste alkyle, alkoxy, alkylthio, formyle, carboxy, alkylcarbonyle, alkoxycarbonyle, trifluorométhyle, un halogène, un groupe cyano, hydroxy ou nitro,

ou l'un de ses sels, de ses produits de solvatation ou des produits de solvatation de ses sels.

**2.** Composé suivant la revendication 1, **caractérisé en ce que**

.... désigne une liaison simple ou une liaison double,
$R^1$ représente l'hydrogène, un reste alkyle en $C_1$ à $C_6$, alkoxy en $C_1$ à $C_6$, alkylthio en $C_1$ à $C_6$, le fluor ou le chlore,
$R^2$ représente l'hydrogène, un reste alkyle en $C_1$ à $C_6$, alkoxy en $C_1$ à $C_6$, alkylthio en $C_1$ à $C_6$, le fluor ou le chlore,
$R^3$ représente un reste alkyle en $C_1$ à $C_4$, un groupe cyano, le fluor, le chlore, un groupe nitro ou un reste trifluorométhyle,

ou bien
l'un des restes $R^1$, $R^2$ et $R^3$ représente l'hydrogène, un reste alkyle en $C_1$ à $C_3$, alkoxy en $C_1$ à $C_3$, un groupe cyano, un halogène, un groupe nitro ou un reste trifluorométhyle, et les deux autres forment, conjointement avec les atomes de carbone auxquels ils sont liés, un noyau cyclopentane ou un noyau cyclohexane,

$R^4$ représente l'hydrogène,
$R^5$ représente l'hydrogène, un reste alkyle en $C_1$ à $C_6$, alkoxy en $C_1$ à $C_6$, carboxy, (alkyle en $C_1$ à $C_6$)-carbonyle, (alkoxy en $C_1$ à $C_6$) carbonyle, trifluorométhyle, le fluor, le chlore, le brome, un groupe cyano, hydroxy, amino, un reste alkylamino en $C_1$ à $C_6$ ou un groupe nitro,
$R^6$ représente l'hydrogène, un reste alkyle en $C_1$ à $C_6$, alkoxy en $C_1$ à $C_6$, carboxy, (alkyle en $C_1$ à $C_6$)-carbonyle, (alkoxy en $C_1$ à $C_6$)carbonyle, trifluorométhyle, le fluor, le chlore, le brome, un groupe cyano, hydroxy, amino, un reste alkylamino en $C_1$ à $C_6$ ou un groupe nitro,

ou bien

$R^5$ et $R^6$ forment un groupe 1,3-dioxolanne conjointement avec les atomes de carbone auxquels ils sont liés,
$R^7$ représente l'hydrogène ou un reste méthyle,
$R^8$ représente un reste alkyle en $C_1$ à $C_3$, alkoxy en $C_1$ à $C_3$, carboxy, aminocarbonyle, (alkyle en $C_1$ à $C_3$)-aminocarbonyle, trifluorométhyle, le fluor, le chlore, un groupe cyano, hydroxy ou nitro,
$R^9$ représente l'hydrogène, un reste alkyle en $C_1$ à $C_3$, alkoxy en $C_1$ à $C_3$, le fluor, le chlore, un groupe cyano ou hydroxy,
et
$R^{10}$ représente l'hydrogène, un reste alkyle en $C_1$ à $C_3$, alkoxy en $C_1$ à $C_3$, le fluor, le chlore, un groupe cyano ou hydroxy.

**3.** Composé suivant l'une des revendications 1 ou 2, **caractérisé en ce que**

.... désigne une liaison simple ou une liaison double,
$R^1$ représente l'hydrogène, un reste méthyle, méthoxy, méthylthio, le fluor ou le chlore,
$R^2$ représente l'hydrogène,
$R^3$ représente un reste méthyle, un groupe cyano, le fluor, le chlore, un groupe nitro ou un reste trifluorométhyle,
$R^4$ représente l'hydrogène,
$R^5$ représente l'hydrogène, un reste méthyle, méthoxy, le fluor ou le chlore,
$R^6$ représente l'hydrogène, un reste méthyle, méthoxy, le fluor ou le chlore,
$R^7$ représente l'hydrogène,
$R^8$ représente un groupe aminocarbonyle, le fluor, le chlore, un groupe cyano ou hydroxy,
$R^9$ représente l'hydrogène,
et
$R^{10}$ représente l'hydrogène.

**4.** Composé suivant l'une des revendications 1 à 3, **caractérisé en ce que** ..... désigne une liaison simple.

**5.** Composé suivant l'une des revendications 1 à 4, **caractérisé en ce que** $R^1$ représente l'hydrogène, un reste méthyle, méthoxy ou le fluor.

**6.** Composé suivant l'une des revendications 1 à 5, **caractérisé en ce que** $R^1$ représente un reste méthoxy.

**7.** Composé suivant l'une des revendications 1 à 6, **caractérisé en ce que** $R^1$ est lié au noyau phényle en position ortho par rapport au site de liaison du noyau phényle.

**8.** Composé suivant l'une des revendications 1, 2 ou 4 à 7, **caractérisé en ce que** $R^2$ représente l'hydrogène.

**9.** Composé suivant l'une des revendications 1, 2 ou 4 à 8, **caractérisé en ce que** $R^3$ représente un reste trifluorométhyle, le chlore, un reste méthyle, isopropyle ou tertiobutyle.

**10.** Composé suivant l'une des revendications 1 à 9, **caractérisé en ce que** $R^3$ représente un reste trifluorométhyle, le chlore ou un reste méthyle.

**11.** Composé suivant l'une des revendications 1 à 10, **caractérisé en ce que** $R^1$ est lié au noyau phényle en position ortho par rapport au site de liaison du noyau phényle et $R^3$ est lié au noyau phényle en position méta à l'opposé de $R^1$ par rapport au site de liaison du noyau phényle.

**12.** Composé suivant l'une des revendications 1 ou 4 à 11, **caractérisé en ce que** $R^4$ représente l'hydrogène.

**13.** Composé suivant l'une des revendications 1, 2 ou 4 à 12, **caractérisé en ce que** $R^5$ représente l'hydrogène, un reste méthyle, méthoxy, le fluor ou le chlore.

**14.** Composé suivant l'une des revendications 1 à 13, **caractérisé en ce que** $R^6$ représente l'hydrogène, un reste méthyle, méthoxy ou le fluor.

**15.** Composé suivant l'une des revendications 1, 2 ou 4 à 14, **caractérisé en ce que** $R^7$ représente l'hydrogène.

**16.** Composé suivant l'une des revendications 1 à 15, **caractérisé en ce que** $R^8$ représente le fluor.

**17.** Composé suivant l'une des revendications 1, 2 ou 4 à 16, **caractérisé en ce que** $R^9$ représente l'hydrogène.

**18.** Composé suivant l'une des revendications 1, 2 ou 4 à 17, **caractérisé en ce que** $R^8$ représente l'hydrogène, un reste méthyle ou le fluor.

**19.** Procédé de production d'un composé de formule (I) suivant la revendication 1, **caractérisé en ce que**, selon le procédé [A], un composé de formule

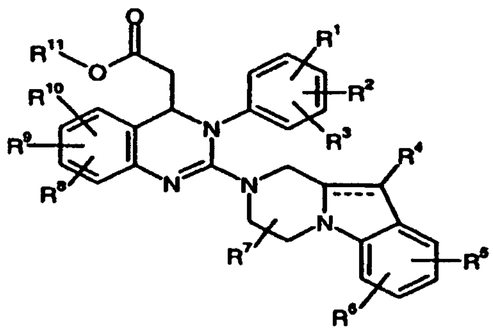

(II),

dans laquelle
....., $R^1$, $R^2$, $R^3$, $R^4$, $R^5$, $R^6$, $R^7$, $R^8$, $R^9$ et $R^{10}$ ont la définition indiquée dans la revendication 1, et $R^{11}$ représente un reste alkyle, avantageusement, le reste méthyle ou éthyle,
est amené à réagir avec des bases
ou bien,
selon le procédé [B], un composé de formule

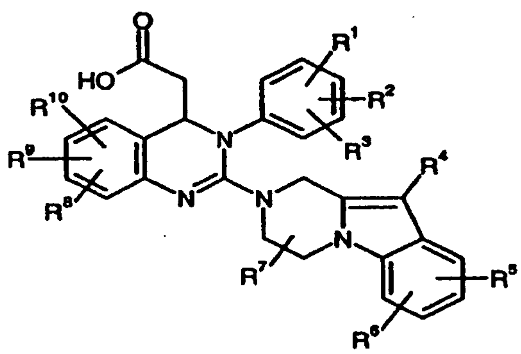

(Ib),

dans laquelle
$R^1$, $R^2$, $R^3$, $R^4$, $R^5$, $R^6$, $R^7$, $R^8$, $R^9$ et $R^{10}$ ont la définition indiquée dans la revendication 1,
est transformé par réaction avec des agents réducteurs en composés de formule

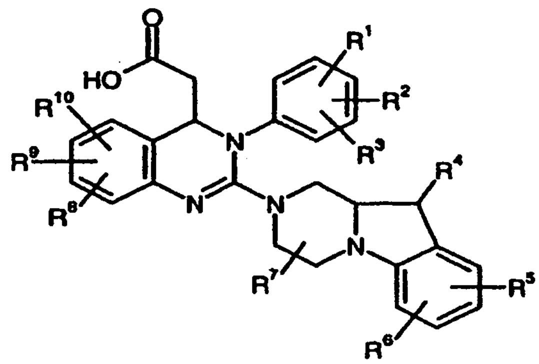

(Ic),

dans laquelle
$R^1$, $R^2$, $R^3$, $R^4$, $R^5$, $R^6$, $R^7$, $R^8$, $R^9$ et $R^{10}$ ont la définition indiquée dans la revendication 1.

**20.** Composé suivant l'une des revendications 1 à 18, destiné au traitement et/ou à la prophylaxie de maladies.

**21.** Médicament contenant un composé suivant l'une des revendications 1 à 18, en combinaison avec une substance auxiliaire inerte non toxique pharmaceutiquement acceptable.

**22.** Utilisation d'un composé suivant l'une des revendications 1 à 18, pour la préparation d'un médicament destiné au traitement et/ou à la prophylaxie d'infections virales.

**23.** Utilisation suivant la revendication 22, **caractérisée en ce que** l'infection virale est une infection par le cytomégalovirus humain (HCMV) ou par un autre représentant du groupe des Herpesviridae.

**24.** Médicament suivant la revendication 21, destiné au traitement et/ou à la prophylaxie d'infections virales.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1201765 A


### In der Beschreibung aufgeführte Nicht-Patentliteratur


- **SAITO T. et al.** *Tetrahedron Lett.,* 1996, vol. 37, 209-212
- **WANG F. et al.** *Tetrahedron Lett.,* 1997, vol. 38, 8651-8654
- **J. CHANG-FONG et al.** *Bioorg. Med. Chem. Lett.,* 2002, vol. 12, 155-158
- **Y. NAKAMURA et al.** *Org. Lett.,* 2002, vol. 4, 2317-2320
- **L.D. BASANAGOUDAR et al.** *Ind. J. Chem.,* 1991, vol. 30B, 1014-1017
- **S.B. RAJUR et al.** *Ind. J. Chem.,* 1989, vol. 28B, 1065-1068
- **A.C. CHENG et al.** *J. Org. Chem.,* 1982, vol. 47, 5258-5262
- **J. CINATL et al.** *FEMS Microbiology Reviews,* 2004, vol. 28, 59-77
- **A. MADAN et al.** Drug-Drug Interaction. *Drugs and the Pharmaceutical Science,* 2002, vol. 116